# EUROPEAN PATENT APPLICATION

(11) **EP 3 892 282 A1**
(43) Date of publication of application: **13.10.2021**
(21) Application number: 19894341.7
(22) Date of filing: 04.12.2019
(51) Int. Cl.: A61K 31/7088, A61K 31/7105, A61K 45/00, A61K 48/00, A61P 35/00, A61P 35/02, A61P 43/00, C12N 15/113

(54) **COMBINATION FOR TREATING CANCER**

(30) Priority: 05.12.2018 JP 2018228283
(71) Applicant: NITTO DENKO CORPORATION, Ibaraki-shi Osaka 567-8680 (JP)
(72) Inventor: TANAKA Hiroyuki, Ibaraki-shi, Osaka 567-8680 (JP); MINOMI Kenjiro, Ibaraki-shi, Osaka 567-8680 (JP); TAKAHASHI Hirokazu, Ibaraki-shi, Osaka 567-8680 (JP); TAMURA Yasuaki, Sapporo-shi, Hokkaido 060-0808 (JP); TAKEI Norio, Sapporo-shi, Hokkaido 060-0808 (JP); YONEDA Akihiro, Sapporo-shi, Hokkaido 060-0808 (JP)
(74) Representative: Hoffmann Eitle
(86) International application number: PCT/JP2019/047504
(87) International publication number: WO 2020/116536

(57) **Abstract**

The present invention relates to a combination comprising (1) a molecule comprising the nucleotide sequence of SEQ ID NO: 1 and (2) an anticancer agent; and a method for treating a cancer, comprising administering an effective amount of the combination to a subject in need thereof.

## Description

### TECHNICAL FIELD

The present disclosure relates to a combination useful for the treatment of a cancer, a method for treating a cancer using the combination, etc.

### BACKGROUND ART

Cancer chemotherapy started with use of drugs, such as alkylating agents, which exhibit cytotoxicity in a cell-non-specific manner. In recent years, molecular targeted drugs have drawn interest which target molecules that are unique to cancers and have smaller influence on cells other than cancer cells. In these circumstances, it has been reported that BcL-XL is upregulated in some types of cancers (Non-Patent Literature 1).

### CITATION LIST

### Non-Patent Literature

Non-Patent Literature 1: Sarosiek and Leta, FEBS J. 2016;283(19):3523-3533

### DISCLOSURE OF THE INVENTION

### PROBLEMS TO BE SOLVED BY THE INVENTION

There is a demand for therapeutic drugs for cancers having small influence on cells other than cancer cells.

### SOLUTION TO PROBLEM

Some aspects of the present disclosure relate to the following.
[1] A combination comprising (1) a molecule comprising the nucleotide sequence of SEQ ID NO: 1 and (2) an anticancer agent.
[2] The combination according to [1], wherein the anticancer agent is an inhibitor of a BcL2 family molecule.
[3] The combination according to [2], wherein the inhibitor of a BcL2 family molecule is an expression inhibitor of the BcL2 family molecule or a function inhibitor of the BcL2 family molecule.
[4] The combination according to [3], wherein the BcL2 family molecule is BcL-XL.
[5] The combination according to any one of [1] to [4], wherein the molecule (1) suppresses the expression of a gene comprising a nucleotide sequence having complementarity to the nucleotide sequence of SEQ ID NO: 1.
[6] The combination according to any one of [1] to [5], wherein the molecule (1) is a miR-345-related molecule selected from double-stranded mature microRNA, pre-microRNA, pri-microRNA, and microRNA mimic of miR-345, and a vector for the expression of one or more thereof.
[7] A pharmaceutical composition comprising a combination according to any one of [1] to [6] and a pharmaceutically acceptable additive.
[8] The combination according to any one of [1] to [6] or the pharmaceutical composition according to [7] for the treatment of a cancer.
[9] The combination or the pharmaceutical composition according to [8], wherein the cancer expresses BcL-XL.
[10] The combination or the pharmaceutical composition according to [8], wherein the cancer is selected from the group consisting of brain tumor, head and neck cancer, breast cancer, lung cancer, oral cancer, esophageal cancer, stomach cancer, duodenal cancer, appendix cancer, colon cancer, rectal cancer, liver cancer, pancreatic cancer, gallbladder cancer, bile duct cancer, anal cancer, kidney cancer, ureteral cancer, bladder cancer, prostate cancer, penile cancer, testicular cancer, uterine cancer, cervical cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and leukemia.
[11] A method for treating a cancer, comprising administering an effective amount of a combination according to any one of [1] to [6] and [8] to [10] or a pharmaceutical composition according to any one of [7] to [10] to a subject in need thereof.
[12] A pharmaceutical composition for treating a cancer in combination with an anticancer agent, the pharmaceutical composition comprising a molecule comprising the nucleotide sequence of SEQ ID NO: 1.

### ADVANTAGEOUS EFFECTS OF INVENTION

The combination and/or the method for treating a cancer using the combination according to the present disclosure are capable of exerting one or more of the following effects according to the aspects.
(1) Can suppress the proliferation of cancer cells.
(2) Can induce the apoptosis of cancer cells.
(3) Have small influence on normal cells.
(4) Have higher anticancer activity than that of a BcL-XL inhibitor alone.
(5) Have higher anticancer activity than that of miR-345 alone.
(6) Can exert an anticancer effect even on a cancer resistant to a BcL-XL inhibitor.
(7) Can exert an anticancer effect even on a cancer resistant to miR-345.

### BRIEF DESCRIPTION OF DRAWINGS

Figure 1 is a diagram showing (A) the enhanced ability to suppress cell proliferation and (B) the ability to induce apoptosis by concurrent use of a BcL-XL inhibitor (Cpd. A or Compound A) and a miR-345-related molecule (Cpd. B or Compound B).
Figure 2 is a diagram showing the enhanced ability to suppress cell proliferation by a combination of the suppression of BcL-XL expression by a BcL-XL inhibitor (Compound D) with a miR-345-related molecule (Compound B).
Figure 3 is a diagram showing the enhanced ability to suppress cell proliferation by a combination of the overexpression of miR-345 by a miR-345-related molecule (Cpd. F or Compound F) with the administration of a BcL-XL inhibitor (Cpd. A or Compound A).
Figure 4 is a diagram showing the influence, on cell proliferation, of a combination of the suppression of BcL-XL expression by a BcL-XL inhibitor (Compound D) with a molecule comprising a CUGACUC sequence (Compound B) or a molecule comprising no CUGACUC sequence (Compound H).
Figure 5 is a diagram showing the influence, on the cell proliferation of normal cells, of a combination of a BcL-XL inhibitor (Cpd. A) and a miR-345-related molecule (Cpd. B).

### DESCRIPTION OF EMBODIMENTS

All technical terms and scientific terms used herein have the same meanings as those usually understood by those skilled in the art, unless otherwise specified herein. All patents, applications and other publications (including online information) cited herein are incorporated herein by reference in their entirety.

The present specification encompasses the contents described in the specification and drawings of the Japanese application filed on December 5, 2018 (Japanese Application No. 2018-228283), based on which the priority of the present applications is claimed.

In one aspect, the present disclosure relates to a combination comprising (1) a molecule comprising the nucleotide sequence of SEQ ID NO: 1 (CUGACUC) and (2) an anticancer agent (hereinafter, also referred to as the "combination of the present disclosure").

Examples of the molecule comprising the nucleotide sequence of SEQ ID NO: 1 (hereinafter, also referred to as a "CUGACUC sequence-containing molecule" or a "CUGACUC-containing molecule") include, but are not limited to, a nucleic acid molecule comprising the nucleotide sequence of SEQ ID NO: XX (hereinafter, also referred to as a "CUGACUC sequence-containing nucleic acid molecule" or a "CUGACUC-containing nucleic acid molecule"). In one embodiment, the CUGACUC-containing nucleic acid molecule is an RNA molecule.

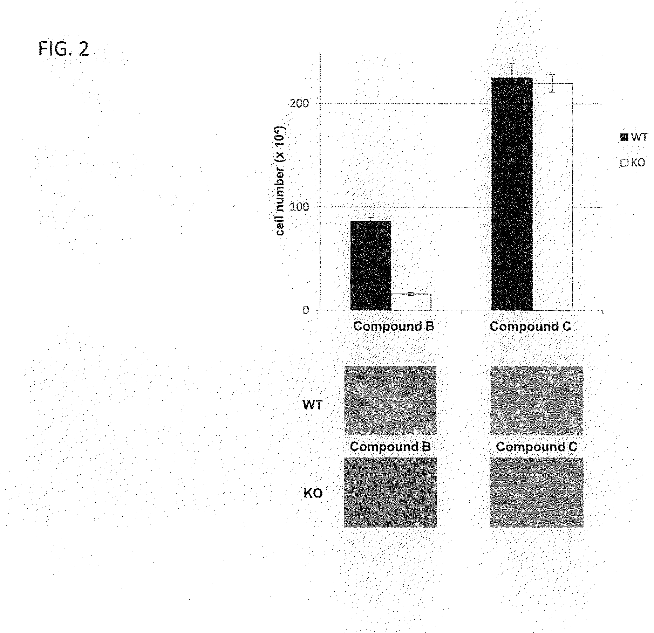

The CUGACUC-containing molecule can preferably suppress the expression of a gene comprising a nucleotide sequence having complementarity to the nucleotide sequence of SEQ ID NO: 1. Such a gene includes, but not limited to, one or more genes selected from, for example, FLG2, AIMP2, TGM1, C12orf76, ZNF133, GPANK1, TMEM139, CTD-2330K9.3, PHF2, SAPCD1, CCL8, HABP4, C6orf164, ARL14, SAT1, RFC1, DNAH10OS, TJP2, KISS1, CNBP, TMEM204, RPS21, ANKRD18A, IL1F10, MBLAC1, GOLGA7B, BRMS1, RSAD2, NMRK1, CYP3A5, IAPP, UBE2L6, AC069547.2, MAP3K7CL, CCDC144NL, CTXN2, GYPE, ZBTB25, RGS1, NOP9, E2F6, HIAT1, PPP2R3A, MRS2, OTX2, TMED7-TICAM2, EPN3, SLC9A3R2, AP000867.1, BST1, RNF4, LIMS3, LIMS3L, C1orf115, NADK, TMEM78, C14orf144, SZRD1, SMAD1, GPR3, C8orf86, SLC2A5, ZNF259, ZNF839, C8orf37, FAM3D, BcL2L11, KLF10, CRTAM, C10orf11, EPB41L2, CDIP1, CXCR5, SLC25A48, HYPK, C20orf202, DUSP18, GJB7, RPA1, KCNA5, PIEZO2, GALR1, PPFIA2, MPEG1, IP6K2, CRISP3, CCDC78, VAPB, SNX12, AIDA, CEACAM8, HFE2, CDKN1A, USP50, FAM57B, OLFM3, CDC42EP3, LILRB1, KRTAP10-11, AC022498.1, MED1, ZNF879, SPARC, IL5, CTTNBP2NL, MCM9, HDGF, PCDH15, TMEM186, PLEKHJ1, RAVER2, PRPSAP1, DST, GAL, LINC00998, SMCP, RPGR, NCMAP, TMCO5A, PBRM1, KRTAP5-2, SLC22A16, FLJ27365, PRSS45, RNPS1, FAM222B, AC093677.1, MRPL36, DRD1, CCBL2, PAX8, FCHO2, RHD, SLC47A2, AKNAD1, C16orf54, CTD-2228K2.5, ZNF14, SLBP, CHCHD5, ARHGAP6, RP11-41O4.1, AACS, CREBL2, SLC35E2B, RTP3, FICD, RTN4, CILP2, LDLRAD3, BLNK, CHRNA1, CCDC136, IKZF1, SLC2A2, TMED4, ZNF598, HMGN1, FAM118A, SNAI3, CENPN, ZNF45, POLR1E, SGMS1, MLC1, IP6K3, RAB27B, AP000322.53, TBX19, C10orf40, RBBP9, SLC9A8, GMEB2, AK4, MTDH, RPP14, SCN10A, DHCR24, SORT1, RAB3B, CDADC1, GATAD1, HSPA12A, CCDC43, NDUFA11, C22orf26, LYRM9, HIST1H3A, S100B, GPR12, APH1A, CPXCR1, DNAJB4, AC007431.1, FSBP, LIG1, TNFSF13B, CASP5, RGL4, B4GALT1, IGFBP4, ALDH1A2, KLRC2, INO80, ZSWIM7, KLRC1, ARL2BP, AIFM1, SNAP23, VTI1A, ATP10B, CYB5D1, VSTM2L, KLK7, GPATCH11, WISP3, GPALPP1, PSMG3, TRIM39, ITPKC, CASS4, SULT1A3, RNF20, SULT1A4, DCP1A, CCDC152, YEATS2, CTC-360G5.8, CLDN18, RGS7, CDA, RTBDN, MTERF, FAM103A1, SELV, C10orf113, KCTD2, NRSN1, ALDOB, SYT4, UBAC2, CDRT1, SRPX2, C17orf96, TFAP2E, CHRDL1, KANK4, IDI2, CTD-2162K18.4, MAN2A2, THY1, IL7R, TBC1D13, NEK7, AL391421.1, C11orf52, GATS, MTF2, CPA4, HEY2, SARS2, FAP, SPOCK3, PAQR7, CDIPT, PDE4B, FAM216B, RHOA, WDR59, LYRM1, CBX7, EZR, ASAP3, TBATA, HERC6, PIP4K2C, PPP2R2A, ELAVL1, HPS5, FLJ00104, TNFRSF10B, CCR5, GJA4, APOC4, LFNG, NOV, C9orf84, ABHD15, ME1, GKN2, NFE2L3, UBL4A, AMIGO1,

TM4SF1, FAM26D, AKT2, SERPINE3, KIAA0895, FBXO31, GPR55, FAM214B, CCDC93, PPAPDC3, XKR4, FGF11, CERKL, FAM53C, DNAJC3, TMEM63B, C14orf177, PTPRN, MOCS2, NAA50, BVES, PRPF3, SMS, C11orf87, SULF2, CYB561, AL589765.1, CDCA8, ZNF396, OSCAR, TCTA, TOR1AIP1, ERLIN2, C12orf66, RUNX3, C22orf46, RP11-80A15.1, CD5, CHST2, GRAPL, CPLX4, RP11-351M8.1, ANAPC2, HLA-DPB1, ALCAM, MXRA5, CARD8, RNF138, TBC1D10C, KCNH1, ERGIC2, SGPP1, MLANA, ERGIC1, NPY4R, SMARCA1, FBXO16, FAM90A1, PRAMEF8, PLB1, PRAMEF7, LRRC20, KIAA1644, AMBP, PCNA, GPX8, OTOS, GBA, WDR31, ZNF689, TMSB15A, MAPK1, INHBE, GLP1R, ZFAND1, MGAT1, GPR157, BcL2, BET1L, SSBP2, TTC19, CD40LG, RAPGEF5, ZNF154, NKAP, FAF2, UBIAD1, WDR70, UAP1L1, PDYN, ZSWIM1, TRIM56, RAD51B, UQCRC2, MUS81, MOCOS, PDE6A, APOBR, OXCT1, METTL9, LYRM7, AQP2, BACE1, TOB1, RASA4B, PRSS8, C2orf50, TMEM86A, C1orf174, DHRS13, CCDC62, MRPL18, SNTN, TNFSF8, NID1, PLA2G12B, FNIP1, KLHL40, ZBTB26, TRIM44, LY6K, SLC39A1, FAM90A26, LINC00923, UHMK1, TMEM33, THAP11, ATF2, AC009365.3, TBCA, NKAIN3, BLMH, CD300E, OR13A1, SPAST, IRF1, FAM65A, GPRC5C, ZNF773, CTD-2368P22.1, FNDC9, ADAM8, DYNLT1, C21orf62, PXN, TXNRD3NB, TNFRSF1B, RPS6KB1, BRCA1, KLHL42, ABCA6, LBR, BMPER, PITRM1, CABLES1, GNPDA2, KRBOX4, TNFRSF13C, SMYD4, BEND4, FLVCR2, GAB2, ITM2A, PPP2R5E, MRPS27, SLC25A18, SYT11, FAM71C, HS3ST3B1, ZNF12, BRS3, SIGLEC9, PPP1R12B, VNN3, TLR5, C8orf47, F11R, METTL21A, CCDC36, SORBS1, GPR98, TINF2, SPRED2, SLC30A3, TMEM168, CLN6, TBX20, ZDHHC12, FP15737, FBLN7, HYAL3, EPS8, PRSS50, MCHR2, PRND, ADORA3, CAPN6, ALPPL2, ARHGAP31, P2RY8, SLC46A1, LAT2, HOXD3, SMIM5, EPHA6, GAN, LYPD1, EIF2B1, PGAM1, KIAA1024L, RP11-10A14.4, TBC1D16, IRAK4, KCNE1, KCTD21, LPAR2, TICRR, FBXO3, LCMT2, FBXL4, DNAH17-AS1, RNFT2, SMIM15, SCN3B, ACTRT3, QRSL1, SEPT2, MRGPRX4, RNF185, VWC2, STX2, GREM1, HTR6, IGFBP3, PLEKHA8, RNF115, AC023632.1, NUP160, TMEM44, TXN2, LDB1, NAA30, PA2G4, AC000003.2, TRAM2, BNIPL, ZMYND12, PRSS33, ZNF844, PEX19, EEF1A1, BUB3, TADA2A, AC132186.1, FANCC, ZNF587, SFTA3, ZSCAN9, MCU, C3orf67, C8orf31, FAM122C, LACC1, RP5-1052I5.2, SLC44A4, PGPEP1, USP25, MS4A1, TRIM24, SDPR, ZKSCAN8, STC2, CDC42SE2, THPO, EPDR1, VWC2L, UMODL1, NSUN7, NFIL3, CHMP4C, SOWAHD, SEPT9, ART4, NAA11, BPIFA2, TRAF1, RAB39B, HSF1, AL079342.1, TRIP12, SNAP29, CCNC, PRAMEF12, PTPN21, ALX4, TFIP11, UBXN10, STON1, RELA, ABCA12, ST8SIA3, PRDM1, CRISPLD2, GPATCH4, MAP2K7, BTNL3, CCDC125, ZNF286B, IL36RN, MORN3, SYTL4, RNF5, NDEL1, FAM83C, CCDC112, CAMK2B, THEGL, RNMTL1, SLC7A6, BTN2A1, AC135983.2, SNX33, GPR75, C19orf40, PLEKHH1, SYPL2, FKBP14, C1orf170, CYBB, RGMA, SECISBP2L,

KIAA1549, SRP19, SLC7A11, SIRPB2, PSMG4, APOA1BP, HSFY1, USP54, AUH, PELI3, NKX6-3, IFNAR1, INSM1, SLC43A3, WDR43, STX3, TFE3, KREMEN1, C1orf192, ABL1, HPCAL4, RAB37, RNF165, HSFY2, RPL27A, NUTM2E, LCN6, N4BP2L1, FAM167A, PRDM2, E2F3, HIF1AN, CCR4, ALDOC, SLCO1A2, NOL12, BBS4, PGAM4, DOK1, ZNF638, CYP1B1, IFIT2, PHYHIP, KCNE3, EHBP1, FAM120AOS, CLEC2D, KITLG, PMP2, PTPN12, NOSTRIN, MATN1, GUSB, CDC23, BBC3, PARP16, FSD2, BSG, ARMC7, ARL5A, WBP2NL, HS3ST5, POC1A, AQP7, SRL, TYSND1, ZMAT3, FCN2, GJB4, IRAK3, PLA2G15, MAP3K1, SLC16A12, GPR112, STARD6, ALS2CR11, RAB33B, PURB, SCRN3, UPK1B, PLCL2, SLC35E1, PTCH1, MMS22L, ADM2, C16orf70, C22orf34, STAMBP, ITIH6, RCN2, ZADH2, FAM210A, NDUFA5, PCDH10, RPL3L, NFKBIB, ZNF48, CELF3, SLC29A2, CLHC1, GPR107, CBL, C3orf70, MMP16, HFE, ACER2, NPEPPS, TEX261, C1orf106, OSM, C17orf72, CKAP4, EIF4E, GABRB3, C12orf4, PRKRIP1, ZEB2, PAH, GPR132, ERVV-1, LPAR1, CCDC28B, SSTR3, ZNF182, SOGA3, RPUSD3, FBLN5, NMUR2, DPH7, KCNN1, STK32B, RAB43, SLC25A19, FAM169A, CLOCK, LRRC38, GCNT4, SLC22A9, ZNF740, USP45, IKZF2, CD83, CACNA1D, MYOZ3, ATOX1, ARHGAP28, C1RL, CHP2, TMEM130, MPZL1, FPR3, CA1, SLCO4C1, TTL, TCP10, AWAT2, EBF2, CBLL1, ZCCHC24, TMPRSS5, C11orf65, CXADR, GRK4, RP9, SETD7, MICAL2, CSRP2BP, ZKSCAN1, SENP5, TSC1, ZNF440, SLC6A20, AIPL1, PPP1R13B, KIAA1279, TMC1, IKBKG, KIAA1737, API5, CHST15, ROPN1, BTNL8, CEACAM7, GM2A, RASL10B, TIGIT, ROPN1B, NT5DC1, C1orf109, TUB, EXO1, DBNDD1, DNA2, MPP2, SLC9C2, NAAA, FAM83F, CLSPN, SLC19A3, HNRNPA3, NUAK1, IL22RA2, RABGAP1L, IDI1, TRAPPC13, SLC25A36, LDLRAD4, CYB561D2, IRF8, OMP, COL1A1, SFRP2, ARG2, PCSK1, TECPR2, PLLP, NAA38, VIPAS39, MAT2B, MFAP3, CDH8, KCNJ1, NPHS2, POLD4, DAP, FAM221B, SHANK3, ROM1, HLA-DOA, TEAD3, APOL4, ELOVL4, BTLA, AKIRIN1, TMEM41A, ZNF135, NOTCH2NL, KMT2C, SLC25A13, ZNF841, GINS2, ZNF768, MRPL19, TSSK6, NLRP10, ITPRIPL2, DNAJC16, USO1, CDC25B, BLOC1S6, SCUBE1, ARSG, ZNF286A, XIRP1, KSR2, FAM175A, TMEM164, CRIM1, RANBP6, MCAM, FFAR2, ZNF525, C11orf21, GALNT10, DCTN5, TMEM201, PTPRF, YWHAE, RAB12, PRDM7, ST8SIA5, CAPN7, WWC3, LIMD2, ZFP3, HOXA3, STAR, UBE2K, DERL1, FMOD, RCSD1, GOLGA6A, FAM19A2, SLC1A2, MINPP1, C7orf63, KIAA1586, RBM28, FAM219A, AL360004.1, PALD1, H2BFM, SLC4A5, MEGF9, FAM47C, ALK, THRB, RNF24, TMEM199, SAMD9, PPP1R16B, SLC1A7, DEC1, TIMELESS, ASB7, PRRG1, SLC2A12, GABARAPL2, GPATCH2L, GNAI3, PIK3R3, OSGIN2, EMP1, SLC22A17, MBP, HEATR6, IRF4, CD48, HTATIP2, ANKH, NDOR1, POLL, PPM1E, SRRD, VDR, C6orf222, AC117395.1, CSRNP3, ATF7IP, ZBTB39, PIAS2, LANCL3, KCNG3, KIAA2018, FLJ20373, ARHGEF37, EPHA5,

ATP6V0E2, DDHD2, ZW10, ABHD2, GLRX3, PPP3R2, ZMYM4, NBPF14, PNN, AKAP12, NT5E, XPC, ETV6, FZD3, TMEM246, GPR161, DNAJC24, UBXN2A, HMX2, RNF222, HIST1H2BN, CCNJL, FAM168A, FRMD6, OPA1, USP46, ISG20, PGRMC2, SLAMF7, P4HA3, SLC23A3, PVALB, AK2, RAPGEF3, PAFAH2, TBC1D5, RS1, TBC1D30, POTEG, GALNTL6, POTEM, GABRA4, SLC16A7, DFFA, SLC30A10, EFNA5, SLC39A11, KIAA1244, MFHAS1, ACSS3, RP11-383H13.1, TRIM10, EXT2, CXorf40B, ZNF215, CDKAL1, MBLAC2, CRISPLD1, UPB1, PAK3, HTR2C, NIPAL1, SEPT11, KCNS1, RGS5, CEP44, CBFA2T2, SPIB, POLD3, TDRD9, CNR2, PHACTR1, VAV2, LMX1B, CDC14A, B3GALT5, NBPF3, AKR1E2, YAP1, DHX57, GSE1, ZNF620, GREM2, PABPC4L, CX3CR1, LRRTM3, LYPLA1, ETV3, EDC4, CPEB3, CCR9, OGFOD2, HPS4, ZCCHC14, NRIP3, IL17RD, ALG10, UVRAG, ZDHHC3, DUOXA1, MED9, AP000695.1, PRRC2C, LILRB4, FBXO25, AC002451.1, IFNAR2, NPR2, COPS5, RPL23, ADIPOQ, CERS3, F2RL3, CYP27B1, BNC1, TRIM62, SV2C, SLC35B4, CXorf56, PIGM, GPR20, KDM2B, STAG2, EPT1, TAB1, GPR42, GIPR, NBPF9, IMPDH1, NGFR, PMPCA, GABRA2, CENPF, CTGF, FSD1L, DPYSL5, BTF3L4, GPR160, BRWD1, LINC00346, SHOX2, HS2ST1, FAM136A, ARL6, PRAMEF18, RHBDD1, RP11-422N16.3, ZNF600, USP33, PRAMEF19, WISP1, TRAPPC11, ORAI2, HLA-E, HECTD2, TCF4, KCNJ15, RUFY4, WNT3, TNFAIP3, FFAR3, WDR64, RETSAT, INHBB, WAC, LACE1, MSANTD3, SERTAD2, DPH3, CD2BP2, ARHGAP26, RNF150, SPATA3, WNT2B, NWD1, GYG2, ZBTB18, RCVRN, EPB41, ZFP14, KCNJ6, CHRNA4, AL356356.1, TRERF1, SFMBT2, ALX3, PVRL1, HS6ST3, SLC25A51, DNAJB12, SLC45A4, CEP104, SPOCK2, ITPR2, KIF5B, RNF125, CYYR1, SOX1, DISCI, NEGRI, UBE2S, VKORC1L1, CCDC14, NCS1, PIRT, LCLAT1, TRPS1, C20orf196, POC1B, CYB561D1, FERMT2, MICALCL, TRIM25, ABCG8, CNTF, RNF144A, C2orf49, CA12, WNT7A, ANKRD13A, DDAH1, KIAA0040, KIAA1239, PLA2G4E, PYCR2, GABRG2, HEBP2, SLC16A10, PRDM6, KIF27, PGBD4, ATXN7L3B, SNX30, SHISA3, SYTL5, NBPF15, PCP4L1, NBPF16, PPP5C, NBPF10, USP32, ALDH1L2, SAMD4B, ZC3H6, TCTE1, DARS2, ARL4D, MAP1LC3A, SPSB4, DKK3, PINK1, RBM8A, GLUD1, AGR2, ARL14EP, FRRS1L, BNIP2, ZNF529, GRB10, PTCD2, ADAMTS4, CCND2, C21orf59, GTF2I, HTR1F, MKLN1, CSMD2, TMEM245, H1F0, MEGF6, SLC17A9, KHNYN, MTHFSD, HOGA1, JPH4, CEP97, TRPM6, RASA4, PRPF38A, GPN2, TARS, SCO1, DRD2, PLXDC1, PRAMEF3, CDR2, FOXO6, KCNS2, USP6, CCDC137, BSND, CERS6, PMEPA1, VEPH1, MTIF2, ITGB3, RAB3D, IRAK2, TMEM155, NUP153, SLC22A15, ZNF621, PDDC1, FOXP1, NDNF, C17orf82, FOXP4, NRG2, SERINC3, SLC25A46, FAM92B, EVI5L, PER2, APOL6, TEX9, SMPD3, LPP, PPP1R9B, RBM20, SPN, PRKCB, CDC42EP2, PATE3, SLC25A47, CATSPER2, POTEI, SYT1, KCNN3, SCD, ZBTB47, NCR3LG1, CHST3, CNPY3, ASAH2B, EXOSC10, SCN1A,

PRAMEF22, RABGAP1, DBNL, PLS3, SNRNP200, XPO4, SLC15A4, EXTL2, ITCH, LRP6, RAB40A, CREB1, OTUD3, JAKMIP2, UBE2J1, DNAJB7, SLC7A1, GPR158, ZFP69B, PTPN18, MYCBP, PANK3, DGKH, ARHGAP11A, OR2W3, RC3H1, C6orf89, SRGAP1, ATP8B4, ZC3H12D, TMTC2, LAMB4, GPR111, PQLC3, RNF144B, ZIC5, GLCE, DCUN1D1, RPL37, TMEM104, LAT, EIF3A, TXLNG, ACVRL1, GPRASP1, SLC8A1, AK7, ENTPD3, CHD2, ARHGEF40, TSPAN9, ODF2L, ZNF652, FADS1, CDC42BPA, CTBP2, BACH2, PRKAB1, ARNT2, PHACTR2, CADM3, FBXL2, ADCYAP1R1, ZNF710, DDX52, ZFYVE20, SP8, C6, PTPRJ, RCAN3, BTAF1, SLC24A4, KIAA0930, PARPBP, PBLD, IFITM5, RP11-17M16.1, TBC1D26, ANKRD22, SASH1, BCOR, VMP1, FAM117B, GOLGA8G, PLCXD1, CHURC1-FNTB, CSGALNACT2, ZRANB3, DYNLL2, MFSD1, RORA, STON2, C1GALT1, ANKRD33B, RND2, SLC9A1, BCL9L, FAM102B, NABP2, SYNGR1, KRIT1, MBTPS2, ZNF415, GPN1, CD58, C7orf61, ONECUT2, RCAN2, DAZAP2, FASTKD2, JPH2, GPSM2, SH2D1B, ST8SIA2, TSPAN6, FAM179B, NHEJ1, ZNF280B, ARHGAP10, RCC2, CHERP, TBC1D4, ARHGAP42, ZNF691, FAM76B, ZNF260, TTC3, TBC1D8B, M6PR, ZNF236, SCUBE3, GTF3C2, KIAA1324, HSPA6, ZNF704, TNR, KLC4, SETBP1, CNDP1, APOL1, FBXW11, LHFPL4, LIPH, RCOR1, TMEM2, PTPRB, ITGB8, FAM126B, ARHGAP22, TAF5, TNFSF14, P2RX1, KANSL3, NRP2, KBTBD6, C1orf158, PLEKHM1, CALN1, TPCN1, NBPF1, JMJD1C, FFAR4, TLL2, DIRAS2, SORD, FAM110C, FAM135B, GRIK3, NBPF20, GRIN2B, GPR37L1, NHS, PFKFB2, CCDC141, IPCEF1, TLCD2, CSGALNACT1, IKZF3, BSN, ADAMTS13, DDX11, SLC7A14, NFASC, NPTXR, NT5C1A, TGM2, LRRC15, AR, WDR65, RP11-770J1.4, XKR7, NPAP1, ZDHHC8, GABBR2, CHD5, ZDHHC15, MKL2, EDEM3, ABR, HOOK3, TTLL12, MLEC, HEG1, RABGEF1, CAMKK2, BHLHE41, H6PD, CLSTN2, AHCYL2, PHIP, OLA1, C20orf112, TAF5L, CLEC5A, RASAL2, SLC25A37, TMEM221, ECM2, NBEAL1, MAPK13, C5orf63, PTPN14, BTN2A2, KAT7, C6orf223, RIOK3, GOLGA3, CYP4F3, PEBP1, MTTP, LRIG2, NBPF11, SMUG1, AC005609.1, GRK1, CHP1, CNIH4, PIGN, RPL10A, ZNF280D, DIO2, THAP1, ZNF490, ZBTB5, COLGALT2, SH2B3, CYP7B1, EMR2, SCIN, TPH1, PRR18, AKR1C2, CD109, HS3ST3A1, PTCD3, MAP9, FAM71F2, NFAT5, EPOR, ZNF19, NICN1, FUT11, ANKRD20A3, TMEM233, USP18, ADCY6, NARG2, AMOTL1, PDE10A, GLIPR2, ZNF451, PDHA1, ERBB3, RDH13, ATG4C, DGKI, KLHDC10, KCNMB3, ANKRD20A4, ARHGEF11, B3GAT2, BLOC1S5, RALGAPB, THSD4, KLHL21, MCHR1, DENND2C, CHL1, ARL2, JAM2, CD59, BMF, PALM2, ME2, ARSB, PPP6R3, COL6A3, EMCN, FBXL3, TNFAIP2, ZNF629, BEND6, SLC45A2, GOLGA6D, GOLGA8J, FAM168B, RPS6, PKN2, HLF, CCDC90B, PCDHB16, NPR1, ACACA, C1QL3, MYO9B, TMEM127, TRIM66, GPR35, SRGAP2, DCAF16, PDK3, QKI, IGF2, PGAP1, ZNF592, C2orf15, MRPL30, ZNF573, PDXK, NUDT5, RAB30, FAM105A,

MED12L, BNIP3, TRPM1, C10orf2, TMEM169, SYN3, C1orf198, MGAT5, SATB1, ATG4B, NSD1, CEP78, RAB22A, CCDC50, GPT2, PPP1R3G, DNAJC18, TMEM242, HS3ST1, APEX2, TMEM231, CTSO, ELF1, IFRG15, CDH19, ANKRD63, UBE2V2, EML1, SCIMP, PRRG3, ZNF202, PTTG1IP, PCGF5, SLC39A9, SYNCRIP, MED8, ANXA11, NOVA2, RNF111, KIAA1147, ZFYVE26, MAN2B2, NCKIPSD, RAB21, MED21, PLXDC2, YIPF4, FLNB, PPP2R5C, SDK2, DGKB, FAIM2, MARVELD1, RASEF, KCNQ3, LRRC8A, ZNF672, GLP2R, MUC6, FAT3, ARHGAP19, RTN3, IL17REL, ARHGAP19-SLIT1, TENM1, ZNF324B, CXorf36, CPLX2, RIC8B, FIGN, DPF2, FKTN, PLXNA2, PAX7, AL627309.1, ZNF445, SLC30A2, TRIM71, SYT6, C10orf105, MED7, KRT74, NTSR1, PLCD3, ERN1, MAFK, PCDH19, NBPF12, SRRM2, INMT, ERC1, SLC24A2, C17orf103, GPR155, TNS1, IQSEC3, CDHR3, NOS1, COL11A2, SNED1, SIPA1L3, TAF8, PIANP, RGS6, CTCF, SHISA6, FIBCD1, PYGB, DAPK1, CMKLR1, ZNF436, KIF1B, SRRM4, PDGFRB, DCAKD, SNX29, KIAA0556, MAPK10, TSPAN33, CLMP, C10orf53, AL590483.1, DBH, PCDH12, IYD, DEPDC5, CENPM, SCN2B, SPEG, KIAA2022, HOXC12, MAPKBP1, FLJ14816, FOXO3, SPECC1L, IFNLR1, TMPRSS4, MYO3B, DAGLA, FBLIM1, PDE11A, CNGA3, FAM155B, AKAP10, TBC1D10B, PDXDC1, ADAM19, TTBK1, TRIM67, FAM222A, ZNF736, PTPN11, SCN4A, ABCC1, C1QTNF7, DNAJC6, PCBP4, CHRNB2, PLEKHN1, SLC9A5, TBX4, GPR56, ARMC5, APC2, STAC, PARD3B, SLC6A17, NDST1, APLN, SAMD12, NOX5, NSG2, EMID1, SPIRE1, CRY2, CD84, PIAS3, IGSF9B, PDE4C, ATP8A1, CRKL, HNF4A, ATP5E, MINOS1-NBL1, NRP1, SHC2, TAPBP, NBL1, FAM83G, HMGN2, P2RY6, TGFB2, MDGA1, PODXL, APOL2, ZBTB34, KMT2D, IQCE, EP400, TTC7A, KCTD15, EGFR, PPP1R10, GXYLT1, CXXC4, FAM196B, RNF11, TRAK1, CCL1, ATP6V1B2, INHBA, ZKSCAN2, IL13RA1, MECP2, EPB41L5, MFN1, KIF26B, USP20, PPP1R3C, QPCTL, NEK9, SNN, NUDCD1, RSPO2, HRH1, LCOR, PCGF2, POU2F1, RAPH1, TANC2, DSTYK, TEF, DENND5B, INSR, TMEM107, WARS, NAT8L, P2RY2, POLR2F, STK35, MYEF2, MYO5A, GDPD1, SP1, CCDC88C, SETX, MNT, SPAG9, ZZZ3, ELL, CC2D1B, PLBD2, CLINT1, ZNF850, NSF, SPTBN2, STK17B, LIMK2, TIAL1, TMOD3, CDK6, ZBTB37, BACE2, ITGA1, APH1B, DHX37, PIGL, SAMD4A, ZNF793, ZNF570, GRAMD1B, KLHL3, TBL1X, AAK1, MARCH6, MTMR3, FGF2, USP8, UCHL5, EIF4E3, TMOD2, TLK1, CACNB4, DNAH11, SCRG1, FAHD1, GIMAP6, NKPD1, PTPN4, EXOC2, PSD3, MRPS36, ZNF431, DYNC1LI2, EBF3, SURF6, FAM179A, LSAMP, AL117190.3, TMED8, TAS2R5, TIMP4, ZNF417, KLF9, ZNF20, ZNF749, TLN2, ZBTB8B, TMEM120B, CHRM3, TREM1, GOLGA6C, PDE8B, MGLL, CDAN1, E2F2, ANKRD12, RALGPS2, WSCD1, ACSF3, KIAA0368, SHE, TOLLIP, SLFN5, HIP1, TIGD6, EFCAB6, G3BP2, UMPS, UACA, AP1S3, TAF9B, GNA12, FSTL4, FAM120B, MTL5, USP40, KCNH5, SRI, NAPEPLD,

RFX7, CRCP, GOLGA8H, GBP4, SMAD3, LOH12CR1, ATP6V0A2, ATP8A2, YOD1, FOSL2, KATNAL1, C7orf60, ANKRD46, KLF3, HELZ, RNF216, PI4K2B, TCEANC2, HGSNAT, CLUAP1, TBX5, HDAC4, GPRC5B, MOB3B, MYO18A, STARD13, LL22NC03-63E9.3, PTCHD4, FAM199X, USP14, LOX, DLC1, FASTKD1, BMPR1A, AASS, TUBB, AQR, RFX3, TBC1D20, DNAJB5, TCHP, TMEM108, ARID3A, KIAA1033, NOL11, C11orf54, BAALC, NR2C2, TATDN3, C9orf69, FOXN4, ZNF667, USP9Y, GLRA2, TCF19, ZNF497, SLC19A1, ST13, KIF6, ID4, C17orf51, STX11, KIAA0141, AGO1, DYRK3, ABT1, TRPV3, DUSP7, UCK2, UBE2J2, AC007040.11, RNF19B, PPM1A, BMP8A, ZBTB21, ZNF696, COL13A1, CCNL1, DBT, TLX1, NOL6, TEX14, CDCP2, MPRIP, LRP8, STXBP5, MAVS, NSFL1C, USP15, ATP5F1, HHIPL1, AP5M1, NUDCD2, CENPA, TIAF1, DNAJB13, RPL37A, CSTF1, PSMF1, MYSM1, CEP350, ZBTB17, LPHN3, ST3GAL1, RBM12, PDIK1L, GOLGA8A, PLEKHM3, PHF7, SFMBT1, KLHL24, SGIP1, ZFYVE28, ACSL3, FAM81A, SRSF7, FAM46C, DTNA, CLMN, SLC20A2, PLXNA1, POT1, ARHGAP20, CBX6, FPGT, YES1, C17orf75, FGD4, TRPM7, SSH3, PHKA2, PCDH18, IL1RAPL1, DCPS, SBSPON, ARMCX3, CDKN2AIP, RAB3IP, TBL3, GOLGA8B, FCRL5, AC137932.1, KIAA0513, FER, ST6GAL2, ADAMTS15, PARP14, ULBP1, SLC26A9, GFI1B, SOCS5, SLC10A2, PPM1M, GSTM4, GPATCH2, NTRK2, TNS3, ZNF365, CYS1, MYOID, XCR1, C170rf102, STARD7, ARHGEF9, XKR8, DRP2, KCNA1, ALS2, GOLGA6L10, THSD7A, CYTH1, KRT80, SIAH3, MARCH1, KRT77, RP11-247C2.2, MOB1B, LRRC10, CCPG1, POLR3B, ABCC12, ZNF37A, ANKRD34B, LAX1, EBF4, MARCH9, TEX22, SLC9A3R1, PDGFRA, PLEKHG4B, ERVFRD-1, SYT10, WBP1L, CA13, KIAA0895L, GOLGA8F, ZNF384, SMG5, NCAN, CD209, TAOK1, CATSPERG, ZNF28, ZNF654, NXPE3, GOLGA8N, KIAA1024, CELSR3, FAM98A, LRRC3DN, FZD8, CACNA1E, DNMT3A, ABCF2, WDR52, LZTS1, AMMECR1, FNTB, PAX5, ARHGAP17, FLYWCH1, SLC30A8, TRIM37, CDC42BPB, BAZ2A, FLT1, MDGA2, CDH5, ZNF681, KCNAB2, DNMBP, GPRIN2, DCHS2, TRIO, HLTF, GOLGA6L9, GOLGA8O, FCF1, KCNMB1, TNFSF15, ISY1-RAB43, EREG, GOLGA8M, CCNF, KCNC1, ABCA13, CCDC144A, DPP10, ZFR2, GNE, FOXI2, EPS15, PIK3IP1, SNX19, WNK2, RGS7BP, ANKS1A, KIAA1429, BMP3, MFSD6, CASP14, ZNF594, CNIH3, LCP2, PRLR, LPAR4, IRGQ, TRIM68, TCEANC, AGO4, ALDH1L1, ASAH2, GOLGA8I, GABRG1, CDR2L, PROSC, WNT9A, RGS12, CRMP1, PLEKHA2, PLCL1, OCLN, IFNGR2, UTS2, JARID2, PRX, FAM63B, KIAA0430, CADM2, ZBTB46, ZNF609, C22orf29, DSC1, ZNF546, GRIA1, EXOC6B, ZFP41, DRAXIN, SLFN11, TEX15, SPHKAP, RHOBTB3, C10orf90, EMC10, EFCAB13, LIPN, RASSF8, FAM65B, TMEM234, TRIM2, LZTS2, TUBB6, GFRA2, PREPL, FAM102A, N4BP2, ZNF660, PRKG1, BMP2K, MOGAT2, CAPZA2, VSTM5, RP13-996F3.5, PTGER3, CCL22, FBXL7, KIF3C, RIPK1, GBF1, GPR173, ARID4A,

CTNNA3, RBM22, NFIA, KCNJ3, SLC36A2, PRKACA, EIF5, ARHGAP32, TDRD6, KIF21A, SLC46A3, POU6F2, KIF14, ATRN, FOXP2, ICAM1, NUP98, ADAM28, SLC39A14, LHX6, GRM5, SCARF1, HCFC1, ZFYVE1, GOLGA8R, FA2H, ATE1, C3orf58, LUZP2, MGAT3, STX1A, KCNA6, ZNF75D, CD96, ABCG1, GOLGA8K, ANKRD62, SPATA13, KLF7, RAB31, PHF17, BHLHE40, ZXDA, CCNI, ETV5, FGFR1, STARD10, ARFGEF2, MAFF, CPD, FAM129A, GPR64, AFF4, ZRANB1, GPR126, TMEM123, SGK1, ALG10B, CYP20A1, TEX30, MAPK14, LHX4, PAICS, GPR50, GIGYF2, SLC7A2, FAM8A1, HNRNPA1, DOCK4, LRRC3, ZXDC, BTD, ABL2, DSC3, ZKSCAN3, CSTF2T, ELP5, PRKCI, TPM3, RAP2A, MTF1, STOX2, PDK1, CDK14, CCNY, RIMKLA, DENND5A, FAR1, KPNA4, IGFBP5, LRRC8B, DCBLD2, ACER3, TRIM14, TBC1D19, GMEB1, EOGT, EDEM1, SDC2, CMTM4, GID4, RNGTT, COMT, CD2AP, STEAP3, TRIM58, IDS, ETF1, CELF1, LGALS8, TFDP2, GMPS, SP3, ISPD, POLR3D, TMTC3, SPRTN, TRIM38, TGOLN2, ADD2, GJA9, KIAA1958, ADIPOR2, CLDN19, STX4, MAGI2, COL8A1, PTAR1, STXBP1, C21orf2, MCTP2, GNB4, ZNF516, TMTC1, FAM126A, ERAP2, MCFD2, MCC, RALGPS1, METTL8, C7orf73, BTBD7, CHDH, MAOA, ZNF321P, ARNTL2, CNR1, CDS2, KIF1A, FLRT2, TMEM98, NUDT16, RBBP4, GPLD1, VWDE, AHRR, MAP3K9, AP5B1, ACAP2, SMARCC1, AP3D1, ENTPD7, TUSC3, AGPAT5, CTBS, SH3BP2, GDI2, ZNF446, EXPH5, MAP2K6, SRA1, SMIM8, WDR41, ZNF562, PAGR1, ZNF10, TSPAN18, PPP4R1L, PARD6G, PPP1R11, KPNA6, ZNF224, FAM212B, CLCN4, SARM1, NFATC2IP, RAB11FIP4, WIPF2, LRRC27, RCL1, C16orf58, CDYL2, TTC9, NDUFAF3, NAB1, DAG1, PSMD11, TRIM72, ZMIZ2, RBBP5, NUP155, KIAA0319, PIFO, RAB3C, USP13, TYW5, SP110, TMEM19, GGA2, TMCC1, KIF11, PPARA, DCAF5, MTAP, TMX1, PRR3, C1orf95, ATXN3, GPD2, NHLRC2, LUZP1, SLC38A1, ZNF324, MPC2, PCYOX1, CORO2A, ENTPD1, PURA, REEP5, ATP6V1C1, INTU, DNAJC5, SLC48A1, HGFAC, UGGT1, PSME3, ILDR2, ALDH3A2, FAM3A, FASTKD5, TRAPPC9, ARIH1, SH3KBP1, RXRB, ATP6V1C2, AGPAT6, FNIP2, AFF2, SIT1, SLC5A12, RBMS2, ORC4, TMEM56, OSBPL3, TPST2, SLC23A2, TNFAIP8, SH2D4A, CAPN5, DNAL1, ZSCAN16, GPR39, MUC13, PHF20L1, GTDC1, SLC9A7, KCNJ5, TIFA, DCP2, PTEN, ISCA2, USP47, SNX2, NLGN2, PDE12, GGACT, NXPH3, PTPRD, CCDC153, SLC1A1, ZC3H8, VAMP4, OARD1, MLXIP, USP6NL, C11orf58, CFD, CLN8, TRPM3, NOA1, EIF2AK2, FNDC3B, C18orf21, AGO3, TLR4, TMEM106B, LNPEP, TENM4, TMC7, XRRA1, FZD5, MXRA7, ZNF514, RAB1A, DDHD1, BHMT2, HNRNPUL2, SCAND3, CLYBL, TMEM200C, DGCR6L, HIST1H2BD, TMEM55A, NRDE2, INSL4, TBC1D1, KCTD20, MAP1LC3B, RBBP8, PHEX, COPZ1, MAPRE2, ANKRD9, C20orf144, SEMA3A, SRCAP, ANKRD49, CECR2, PLAC8, ADARB2, CD55, DCLK1, AVPR1A, GMPPB, R3HDM1, ADARB1, SNX16, NSL1, CYLD, FXN, ACTN2, ABI2,

SPPL2A, CENPL, PTBP2, PPM1L, FBXL20, TLN1, P2RX7, MBD3, TFAP2B, PPIP5K2, PRSS21, NDUFS1, CD38, FRS2, RP11-47I22.3, PTCHD1, MRPL2, CISD1, USP37, MFSD9, SPATA33, ZKSCAN4, HAUS5, ZBTB40, KIAA1045, FAM120C, CTNND1, SF3A1, EFCAB11, UBA6, ICK, TMEM220, HSDL2, AFF1, CTH, FAM105B, NCOR1, GNL1, ZNF805, FRMD4A, SFT2D3, CTNNBIP1, ARRB1, NECAB3, GCNT1, RAB14, CCDC77, EIF4EBP3, TXNL1, UPP1, PDE4DIP, FRK, ZNF468, ESYT2, RPL13, MSH5, HEXA, MCMBP, HSPA4L, MLH3, PRMT3, SGPL1, GGCX, UQCC2, FAM104A, SLC33A1, UBE2T, ERBB2IP, FBXW2, QDPR, CCSER2, WDR33, ADAMTS5, CKAP2L, KIAA1377, ZNF701, PPP1R12A, IL20RA, FLT3, ZNF117, ERG, ENDOD1, ZCCHC9, FAM26E, MXD1, GPR180, FGFR1OP, CEP57L1, INSIG2, CCDC149, TNPO3, RNF170, SMC2, OTUD7B, SENP1, MBD2, ANTXR1, PDHX, MRGPRF, N4BP3, TMEM110, ARFGEF1, C11orf74, DCAF8, RYR2, AK1, STRIP1, TFCP2L1, C19orf70, TAF12, WDFY2, TMBIM4, NEDD4L, MFAP4, C3orf62, PKIA, ERCC6, GNB1L, C11orf48, NAGS, DEPTOR, GDF5OS, EMC3, LRRC47, MTO1, AP2B1, FUT10, LMOD1, RANBP17, HELLS, RBFA, C3orf14, SLC29A4, PHF21A, TBL1XR1, MRRF, TMEM241, PLEKHG3, MGAT4A, TUSC2, MYPN, VSTM4, SMAP1, RHPN1, RAP2B, WRN, TRIP4, PIK3C3, YTHDC1, FKBP9, WDR37, ACADSB, DOCK5, CBX5, ZNF670, FAM114A2, CXorf23, ACAD11, MCM3AP, ZBTB44, CNKSR3, CREBRF, OSTF1, MAGIX, BOD1, SMAD2, DDX19B, UROS, SFT2D2, MRPS10, DDX18, SOCS7, AKT3, ENSA, SMARCE1, CEP41, TRIM13, ZNF780B, MLPH, SERPINA4, TTC33, PPM1H, FXR1, ALG5, SEPHS1, TTF2, KCTD16, SLC11A2, HNRNPD, C1orf21, RBM12B, CCDC171, MTPAP, FAF1, SLC22A3, TCEB3, C7orf25, CHKA, ANKIB1, ZNF326, WDR3, MRPL3, FAM122A, SKP1, HTRA1, REST, RAB11B, SLC35C2, LRPAP1, INTS8, PON3, XRCC3, CYTH2, RBM23, ZFP62, NGDN, ARFRP1, UBE2R2, THBS2, ATP1B2, SMIM7, C2orf68, RPAP2, PON1, KCMF1, CTSB, TXNDC17, ORC1, PGBD1, MRPL17, METTL14, KDM5B, TRAF4, SUSD1, RPS6KA5, NPC2, FAM151B, PIK3CA, KNTC1, HLA-A, ZNF554, JDP2, ZNF526, HPX, C12orf5, CPE, FOCAD, ASPSCR1, SYT7, CASD1, MRPL51, BTBD9, FTO, LITAF, F10, LYRM4, MAPK6, TMBIM6, PSMB5, TRIM27, PLA2G12A, GLG1, TCF21, FAIM3, BCAS3, C19orf47, RWDD1, TMED7, TSPAN14, RBM4B, PTDSS1, SIK2, HARBI1, KIAA1551, SPICE1, SVIP, GTF2F2, CNGA4, PHKG2, MMAB, CRNKL1, FZD1, QSOX1, ZNF382, RSBN1L, GOSR1, ACSM2A, CNNM2, TMEM184C, BAMBI, GPBP1L1, NCAPH2, C9orf64, FUT4, WNK1, PLEKHO1, RAP1A, WBP11, USP38, CORO7, CRTAP, WBP4, ABRACL, GNG11, PGBD2, YWHAB, F7, LONP2, BNIP3L, SLC22A23, ALG14, ANG, ABCF3, SLCO1B3, ZNF607, GPR65, CDC6, DONSON, ZNF226, LPHN2, MCOLN3, L3HYPDH, ARHGEF5, MED31, CYP2A7, FAM175B, CHML, FHL5, SLC35E2, C14orf119, FUCA2, FARSB, TK2, CHID1, APOPT1, C11orf83, ZNF641, COPS8, KPNA3, MTHFR, ADK,

CCNI2, HLA-DRB5, G6PC2, ZNF860, FXYD5, CNGB1, TWISTNB, PSMB7, GPR34, MAP4K4, CDK13, ANKHD1-EIF4EBP3, CHST12, MSH3, MYL12B, AKR1C4, DYRK4, INPP4A, AKT1, LOXL3, EMILIN2, AKR7A2, ZNF107, RBM15B, PRR13, BPIFA3, ATP2B2, ANXA4, HTT, SGSM2, SND1, ACO1, CHD7, IMMT, PANK2, TACO1, OR2A4, G6PC3, TPBG, NAT10, SLC25A32, KIAA0391, ACSM2B, MAGEB10, SLC35D2, PYCR1, AVPI1, CDK12, CYB5R3, CDX2, C3, DYRK2, PPIL4, SLC25A16, CERCAM, ACAD10, PLRG1, LMBR1, METTL24, VLDLR, PCYT2, SMIM12, RWDD2B, CCDC158, HIF1A, HSBP1, OIP5, B3GAT3, RFFL, SRFBP1, LRRC6, AARD, ITGA11, PPP1CC, RNF126, BAG1, SHB, IGSF3, TRIP13, RYBP, TEPP, DOCK7, LMF1, CEP63, DAPP1, SELM, COLEC10, SOD2, MLIP, GDAP2, POLA2, GLUL, TMEM70, FOXRED2, TSC2, AGTRAP, DDX5, CAD, TPD52L1, DR1, PDCD6IP, GATA6, GNRHR, CAMSAP1, HUWE1, LIMD1, ZNRF2, TAF1B, CKAP5, NR2F6, PPIC, HDAC2, BRD9, NBN, CSTF2, RNPEPL1, GLYAT, DGCR14, RPS12, PRSS23, NKX2-1, GTF2B, ZNF579, TACR2, OR7A5, CABP4, NSUN3, GRK5, NUDCD3, TCP10L, C8A, ST3GAL6, HMGCR, SIGLEC14, TPT1, RNF213, TMEM37, ZNF17, NUP188, AC007952.5, DCAF15, NCF1, MS4A2, CYP2C19, NCKAP1, PFKM, CD300C, ANKRD20A1, RAD51L3-RFFL, ZNF626, RP11-210M15.2, TLR8, LRRC2, SLITRK3, ANKRD20A2, METTL22, TTLL2, UBE2QL1, UGT2B4, GABRR2, STRN, ZBTB20, MSH2, MRPL22, MYO1G, IMPA1, C10orf71, CTD-2545M3.6, FGF5, PAX1, GRIN2A, MGAT4C, C16orf72, TCEAL4, RP11-343C2.12, AC106017.1, PAM, AGAP2, KCNJ13, TLK2, NLRP9, RNF214, GINM1, POLDIP2, SHROOM4, BMP10, HLA-DRB1, CLEC18A, ZNF283, SH3TC2, DNAJC10, MALSU1, FNDC5, FAM127A, TCF23, PATE2, SLC25A11, SLC2A4RG, GABRB1, TMC2, ARL6IP4, HJURP, DIRC2, ANGPT4, PRH2, PIGP, SF3B3, MOBP, TM9SF3, ATG7, NUP37, CYR61, CRP, FSTL1, GTF2H5, CLPP, ALDH2, TM2D2, AGFG2, PPL, NBPF24, GPR25, FBXO47, RCAN1, SLA, CNOT7, GRWD1, PDF, B3GALT2, SELPLG, ELP2, SEC23IP, TMEM150C, PCDH8, PDCL, ATP1A3, PLGLB2, SLC10A6, PLGLB1, OTUB1, BID, KLF2, RGS18, PLA2G16, ERCC6L2, GDE1, KY, LRRFIP1, POPDC2, POTED, KNG1, CCT5, CNTNAP5, GSC2, RP13-996F3.4, CERS5, OS9, C14orf23, ZNF70, EXOSC1, C6orf25, CD226, APOB, ACOT2, ABCD2, BTN3A2, SYNPO2, PSMB9, LRRC40, ELF4, NFIB, METAP1, PTPRC, FPR1, DSE, APC, ZNF8, ENPP6, PIK3CG, TBL2, NKAPL, GOLGA6B, CCNO, RBM14, ELK1, WT1, SC5D, LRRC49, NAP1L4, PANK1, LIPG, CDC73, ANKFY1, SLMAP, NRXN3, CRHR2, AGT, KLHDC8A, CUL4A, IL10RA, CACNA1C, XPNPEP2, DNAJC15, AC002472.13, ATG13, PSMB2, PPDPF, PPP1R15B, RNF187, MRVI1, ATP6V1E1, SART1, DECR1, SLC28A1, DCC, CLU, VEGFB, GUCY1A3, BCKDK, PHF19, HSPA14, CACUL1, STARD3NL, SSTR2, CHIC2, FGF10, CLEC7A, ZBTB7C, SLC25A26, KIAA1715, PSTPIP1, COMMD9, CD180, FGL2, SPATS2L, MMP19, XRCC5, ZBTB16, SHC3, CCDC71, HIST1H2BG, MAG, MAP7, TFAM, NUP93, DCUN1D5, EHD3, PTPRA, DENND2D, GHITM, TYRP1, RUNDC1, FUS, CYP2C8, VASN, PRDM16, AREL1, ATP2B1, CTDP1, C1orf27 and SCARA3.

More preferable gene includes one or more genes selected from FLG2, AIMP2, TGM1, C12orf76, ZNF133, GPANK1, TMEM139, CTD-2330K9.3, PHF2, SAPCD1, CCL8, HABP4, C6orf164, ARL14, SAT1, RFC1, DNAH10OS, TJP2, KISS1, CNBP, TMEM204, RPS21, ANKRD18A, IL1F10, MBLAC1, GOLGA7B, BRMS1, RSAD2, NMRK1, CYP3A5, IAPP, UBE2L6, AC069547.2, MAP3K7CL, CCDC144NL, CTXN2, GYPE, ZBTB25, RGS1, NOP9, E2F6, HIAT1, PPP2R3A, MRS2, OTX2, TMED7-TICAM2, EPN3, SLC9A3R2, AP000867.1, BST1, RNF4, LIMS3, LIMS3L, C1orf115, NADK, TMEM78, C14orf144, SZRD1, SMAD1, GPR3, C8orf86, SLC2A5, ZNF259, ZNF839, C8orf37, FAM3D, BcL2L11, KLF10, CRTAM, C10orf11, EPB41L2, CDIP1, CXCR5, SLC25A48, HYPK, C20orf202, DUSP18, GJB7, RPA1, KCNA5, PIEZO2, GALR1, PPFIA2, MPEG1, IP6K2, CRISP3, CCDC78, VAPB, SNX12, AIDA, CEACAM8, HFE2, CDKN1A, USP50, FAM57B, OLFM3, CDC42EP3, LILRB1, KRTAP10-11, AC022498.1, MED1, ZNF879, SPARC, IL5, CTTNBP2NL, MCM9, HDGF, PCDH15, TMEM186, PLEKHJ1, RAVER2, PRPSAP1, DST, GAL, LINC00998, SMCP, RPGR, NCMAP, TMCO5A, PBRM1, KRTAP5-2, SLC22A16, FLJ27365, PRSS45, RNPS1, FAM222B, AC093677.1, MRPL36, DRD1, CCBL2, PAX8, FCHO2, RHD, SLC47A2, AKNAD1, C16orf54, CTD-2228K2.5, ZNF14, SLBP, CHCHD5, ARHGAP6, RP11-41O4.1, AACS, CREBL2, SLC35E2B, RTP3, FICD, RTN4, CILP2, LDLRAD3, BLNK, CHRNA1, CCDC136, IKZF1, SLC2A2, TMED4, ZNF598, HMGN1, FAM118A, SNAI3, CENPN, ZNF45, POLR1E, SGMS1, MLC1, IP6K3, RAB27B, AP000322.53, TBX19, C10orf40, RBBP9, SLC9A8, GMEB2, AK4, MTDH, RPP14, SCN10A, DHCR24, SORT1, RAB3B, CDADC1, GATAD1, HSPA12A, CCDC43, NDUFA11, C22orf26, LYRM9, HIST1H3A, S100B, GPR12, APH1A, CPXCR1, DNAJB4, AC007431.1, FSBP, LIG1, TNFSF13B, CASP5, RGL4, B4GALT1, IGFBP4, ALDH1A2, KLRC2, INO80, ZSWIM7, KLRC1, ARL2BP, AIFM1, SNAP23, VTI1A, ATP10B, CYB5D1, VSTM2L, KLK7, GPATCH11, WISP3, GPALPP1, PSMG3, TRIM39, ITPKC, CASS4, SULT1A3, RNF20, SULT1A4, DCP1A, CCDC152, YEATS2, CTC-360G5.8, CLDN18, RGS7, CDA, RTBDN, MTERF, FAM103A1, SELV, C10orf113, KCTD2, NRSN1, ALDOB, SYT4, UBAC2, CDRT1, SRPX2, C17orf96, TFAP2E, CHRDL1, KANK4, IDI2, CTD-2162K18.4, MAN2A2, THY1, IL7R, TBC1D13, NEK7, AL391421.1, C11orf52, GATS, MTF2, CPA4, HEY2, SARS2, FAP, SPOCK3, PAQR7, CDIPT, PDE4B, FAM216B, RHOA, WDR59, LYRM1, CBX7, EZR, ASAP3, TBATA, HERC6, PIP4K2C, PPP2R2A, ELAVL1, HPS5, FLJ00104, TNFRSF10B, CCR5, GJA4, APOC4, LFNG, NOV, C9orf84, ABHD15, ME1, GKN2, NFE2L3, UBL4A, AMIGO1, TM4SF1, FAM26D, AKT2, SERPINE3, KIAA0895, FBXO31, GPR55, FAM214B, CCDC93, PPAPDC3, XKR4, FGF11, CERKL, FAM53C, DNAJC3, TMEM63B, C14orf177, PTPRN, MOCS2, NAA50, BVES, PRPF3, SMS, C11orf87, SULF2, CYB561, AL589765.1, CDCA8, ZNF396, OSCAR, TCTA, TOR1AIP1, ERLIN2, C12orf66, RUNX3, C22orf46, RP11-80A15.1, CD5, CHST2, GRAPL, CPLX4, RP11-351M8.1, ANAPC2, HLA-DPB1, ALCAM, MXRA5, CARD8, RNF138, TBC1D10C, KCNH1, ERGIC2, SGPP1, MLANA, ERGIC1, NPY4R, SMARCA1, FBXO16, FAM90A1, PRAMEF8, PLB1, PRAMEF7, LRRC20, KIAA1644, AMBP, PCNA, GPX8, OTOS, GBA, WDR31, ZNF689, TMSB15A, MAPK1, INHBE, GLP1R, ZFAND1, MGAT1, GPR157 and BcL2.

Particularly preferable gene includes one or more genes selected from BcL2 and Smad1.

The suppression of gene expression can be evaluated, for example, by comparing the expression level of the gene between cells with the action of the CUGACUC-containing molecule and cells without the action thereof. The expression level of the gene can be determined by a known detection approach, for example, by detecting a nucleic acid molecule encoding the gene via various hybridization methods, Northern blotting, Southern blotting, or various PCR methods using a nucleic acid specifically hybridizing to the nucleic acid molecule or a unique fragment thereof, or by detecting a protein encoded by the gene via a known protein detection approach, for example, an immunoprecipitation method using an antibody, EIA (enzyme immunoassay) (e.g., ELISA (enzyme-linked immunosorbent assay)), RIA (radioimmunoassay) (e.g., IRMA (immunoradiometric assay), RAST (radioallergosorbent test), and RIST (radioimmunosorbent test)), Western blotting, an immunohistochemical method, an immunocytochemical method, or flow cytometry, without limitations.

In one embodiment, the CUGACUC-containing nucleic acid molecule exhibits a miRNA-like effect or forms a molecule that exhibits a miRNA-like effect. The miRNA-like effect typically refers to sequence-specific degradation or translational suppression of RNA induced by miRNA. This effect includes the mature miRNA-mediated suppression of translation from mRNA complementary to a seed sequence (sequence from positions 2 to 8 from the 5' end), and promotion of the degradation of the mRNA by degrading the 3'-terminal poly-A tail of the mRNA.

The length of the CUGACUC-containing nucleic acid molecule is not particularly limited and can be, for example, a length of 10 to 5000, 12 to 3000, 13 to 2000, 14 to 1000, 15 to 800, 16 to 700, or 17 to 600 nucleotides.

The CUGACUC-containing nucleic acid molecule can be single-stranded or double-stranded, and the CUGACUC sequence moiety may or may not form a duplex. The CUGACUC-containing nucleic acid molecule may be single-stranded, and the CUGACUC sequence moiety may form a duplex. In this case, the CUGACUC sequence may have a 3' or 5' loop, preferably a 3' loop. The CUGACUC-containing nucleic acid molecule may have CUGACUC at positions 2 to 8 from the 5' end.

In a preferred embodiment, the CUGACUC-containing nucleic acid molecule is a miRNA-related molecule selected from single-stranded mature microRNA, double-stranded mature microRNA, pre-microRNA, pri-microRNA, microRNA mimic, and a vector for the expression of one or more thereof. The CUGACUC sequence may reside in the 5' arm (microRNA-5p) or 3' arm (microRNA-3p) of microRNA or may reside in a loop portion (Loop-miR (Winter et al., Nucleic Acids Res. 2013; 41 (10): 5503-12)). In a preferred embodiment, the CUGACUC sequence resides in the 5' arm of microRNA.

The microRNA mimic is a nucleic acid molecule that mimics the function of endogenous microRNA, and is well known in the art (e.g., van Rooij and Kauppinen, EMBO Mol Med. 2014; 6 (7): 851-64; and Chorn et al., RNA. 2012; 18 (10): 1796-804). The microRNA mimic may contain a chemical modification, and its nucleotide sequence may be changed with respect to the endogenous microRNA. The chemical modification may include various modifications mentioned later. The chemical modification preferably has a small adverse effect on the incorporation of a portion (guide strand) containing the CUGACUC sequence of the microRNA mimic into AGO. Non-limiting examples of the chemical modification preferably include 2'-fluoro modification, 2'-O-methyl modification, 2'-O-methoxyethyl modification, LNA, phosphorothioate bonds, morpholino, and PNA. The microRNA mimic may have a nucleotide sequence identical to that of the endogenous microRNA (e.g., pre-microRNA) or may have a nucleotide sequence having the deletion, substitution, or addition of one or more nucleotides in the nucleotide sequence of the endogenous microRNA. The microRNA mimic may be double-stranded or single-stranded. The double-stranded one may have one or more single-stranded portions.

In a particular preferred embodiment, the CUGACUC-containing nucleic acid molecule may comprise the nucleotide sequence of SEQ ID NO: 2 (mature-mir-345-5p), or SEQ ID NO: 3 (pre-mir-345).

In a particular preferred embodiment, the CUGACUC-containing nucleic acid molecule is hsa-miR-345-related molecule selected from single-stranded mature microRNA, double-stranded mature microRNA, pre-microRNA, pri-microRNA, and microRNA mimic of hsa-miR-345, and a vector for the expression of one or more thereof.

The nucleic acid molecule of the present disclosure may comprise an unmodified nucleotide and/or a modified nucleotide. In the present specification, the unmodified nucleotide and the modified nucleotide are simply referred to as a "nucleotide" collectively. The unmodified nucleotide refers to a naturally occurring nucleotide constituting DNA or RNA, i.e., a substance constituted by a nucleobase (adenine, guanine, uracil, thymine, or cytosine), a sugar (ribose or deoxyribose), and a phosphate group. In an unmodified nucleic acid molecule constituted by unmodified nucleotides, the 3' position of one of two adjacent unmodified nucleotides is usually linked to the 5' position of the other unmodified nucleotide through a phosphodiester bond. In one embodiment, the unmodified nucleotide is an unmodified ribonucleotide, and the unmodified nucleic acid molecule is constituted by unmodified ribonucleotides.

The modified nucleotide refers to a nucleotide containing a chemical modification to the unmodified nucleotide. The modified nucleotide may be artificially synthesized or may occur naturally. The modified nucleotide includes a nucleotide modified at its nucleobase, sugar, backbone (internucleotide bond), 5' end and/or 3' end. The modified nucleotide also includes a nucleotide modified at any one of these sites as well as a nucleotide modified at two or more of the sites.

Examples of the modification to the nucleobase include, but are not limited to, 2,4-difluorotoluyl, 2,6-diamino, 5-bromo, 5-iodo, 2-thio, dihydro, 5-propynyl, and 5-methyl modifications, and elimination of a base. Examples of modified nucleobase include, but are not limited to, xanthine, hypoxanthine, inosine, 2-aminoadenine, 6-methyl and other alkyl derivatives of adenine and guanine, universal base, 2-propyl and other alkyl derivatives of adenine and guanine, 5-halouracil and 5-halocytosine, 5-propynyl uracil and 5-propynyl cytosine, 6-azo uracil, 6-azo cytosine and 6-azo thymine, 5-uracil (pseudouracil), 4-thiouracil, 8-halo, amino, thiol, thioalkyl, hydroxyl and other 8-substituted adenines and guanines, 5-trifluoromethyl and other 5-substituted uracils and cytosines, 7-methylguanine, acyclonucleotides, deazapurines, heterocyclic substituted analogs of purines and pyrimidines, e.g., aminoethyoxy phenoxazine, derivatives of purines and pyrimidines (e.g., 1-alkyl-, 1-alkenyl-, heteroaromatic- and 1-alkynyl derivatives) and tautomers thereof, 8-oxo-N6-methyladenine, 7-diazaxanthine, 5-methylcytosine, 5-methyluracil, 5-(1-propynyl) uracil, 5-(1-propynyl) cytosine and 4,4-ethanocytosine, non-purinyl and non-pyrimidinyl bases such as 2-aminopyridine and triazines, abasic nucleotide, deoxy abasic nucleotide, inverted abasic nucleotide, inverted deoxy abasic nucleotide, and the like.

Examples of the modification to the sugar include, but are not limited to: modifications at position 2', for example, 2'-O-alkyl modifications (e.g., 2'-O-methyl modification and 2'-O-ethyl modification), 2'-methoxyethoxy modification, 2'-methoxyethyl modification, 2'-deoxy modification, 2'-halogen modifications (2'-fluoro modification, 2'-chloro modification, 2'-bromo modification, etc.), 2'-O-allyl modification, 2'-amino modification, 2'-S-alkyl modification, 2'-O-[2(methylamino)-2-oxoethyl] modification, 2'-alkoxy modification, 2'-O-2-methoxyethyl, 2'-allyloxy (-OCH₂CH=CH₂), 2'-propargyl, 2'-propyl, 2'-O-(N-methyl carbamate) modification, 2'-O-(2,4-dinitrophenyl) modification, and 2'-deoxy-2'-fluoro-β-D-arabino modification; modifications at position 4', for example, 4'-thio modification and 4'-C-hydroxymethyl modification; and other modifications with ethynyl, ethenyl, propenyl, CF, cyano, imidazole, carboxylate, thioate, C₁ to C₁₀ lower alkyl, substituted lower alkyl, alkaryl or aralkyl, OCF₃, OCN, O-, S- or N-alkyl, O-, S- or N-alkenyl, SOCH₃, SO₂CH₃, ONO₂, NO₂, N₃, heterocycloalkyl, heterocycloalkaryl, aminoalkylamino, polyalkylamino or substituted silyl. Other examples of the modified sugar include locked nucleic acid (LNA), oxetane-LNA (OXE), unlocked nucleic acid (UNA), ethylene-bridged nucleic acid (ENA), altriol nucleic acid (ANA), and hexitol nucleic acid (HNA).

In the present disclosure, alkyl group includes saturated aliphatic groups, including straight-chain alkyl groups (e.g., methyl, ethyl, propyl, butyl, pentyl, hexyl, heptyl, octyl, nonyl, decyl, etc.), branched-chain alkyl groups (isopropyl, tert-butyl, isobutyl, etc.), cycloalkyl (alicyclic) groups (cyclopropyl, cyclopentyl, cyclohexyl, cycloheptyl, cyclooctyl), and alkyl substituted cycloalkyl groups. In certain embodiments, a straight chain or branched chain alkyl has 6 or fewer carbon atoms in its backbone (e.g., C₁-C₆ for straight chain, C₃-C₆ for branched chain), and more preferably 4 or fewer. Likewise, preferred cycloalkyls may have from 3-8 carbon atoms in their ring structure, and more preferably have 5 or 6 carbons in the ring structure. The term C₁-C₆ includes alkyl groups containing 1 to 6 carbon atoms. The alkyl group can be substituted alkyl group such as alkyl moieties having substituents replacing a hydrogen on one or more carbons of the hydrocarbon backbone. Such substituents can include, for example, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

In the present disclosure, alkoxy group includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, etc.

In the present disclosure, halogens include fluorine, bromine, chlorine, iodine.

Examples of modified backbone include, but are not limited to phosphorothioate, thiophosphate-D-ribose entities, triester, thioate, 2'-5' bridged backbone (may also be referred to as 5'-2' or 2'5' nucleotide or 2'5' ribonucleotide), PACE, 3'-(or -5')deoxy-3'-(or -5')thio-phosphorothioate, phosphorodithioate, phosphoroselenates, 3'-(or -5')deoxy phosphinates, borano phosphates, 3'-(or - 5')deoxy-3'-(or 5'-)amino phosphoramidates, hydrogen phosphonates, phosphonates, borano phosphate esters, phosphoramidates, alkyl or aryl phosphonates and phosphotriester modifications such as alkylphosphotriesters, phosphotriester phosphorus linkages, 5'-ethoxyphosphodiester, P-alkyloxyphosphotriester, methylphosphonate and morpholino, and nonphosphorus containing linkages for example, carbonate, carbamate, silyl, sulfur, sulfonate, sulfonamide, formacetal, thioformacetyl, oxime, methyleneimino, methylenemethylimino, methylenehydrazo, methylenedimethylhydrazo and methyleneoxymethylimino linkages.

Examples of 5'- and/or 3'- end modification include addition of a capping moiety to 5'- and/or 3'- end, and modification at terminal phosphate groups, such as [3-3']-inverted deoxyribose, deoxyribonucleotide, [5'-3']-3'-deoxyribonucleotide, [5'-3']-ribonucleotide, [5'-3']-3'-O-methyl ribonucleotide, 3'-glyceryl, [3'-5']-3'-deoxyribonucleotide, [3'-3']-deoxyribonucleotide, [5'-2']-deoxyribonucleotide, and [5-3']-dideoxyribonucleotide. Non-limiting examples of capping moiety include an abasic nucleotide, a deoxy abasic nucleotide, an inverted (deoxy) abasic nucleotide, a hydrocarbon (alkyl) moiety and derivatives thereof, a mirror nucleotide (L-DNA or L-RNA), bridged nucleic acids including LNA and ethylene bridged nucleic acids, linkage modified nucleotides (e.g. PACE) and base modified nucleotides, glyceryl, dinucleotide, acyclic nucleotide, amino, fluoro, chloro, bromo, CN, CF, methoxy, imidazole, carboxylate, thioate, C₁ to C₁₀ lower alkyl, substituted lower alkyl, alkaryl or aralkyl, OCF₃, OCN, O, S , or N-alkyl, O, S , or N-alkenyl, SOCH₃, SO₂CH₃, ONO₂, NO₂, N₃, heterocycloalkyl, heterocycloalkaryl, aminoalkylamino, polyalkylamino or substituted silyl. The capping moiety may serve as a non-nucleotide overhang.

Modified nucleotides of the present disclosure include 2'-deoxyribonucleotides, 2'-O-methyl ribonucleotides, 2'-deoxy-2'-fluoro ribonucleotides, universal base nucleotides, acyclic nucleotides, 5-C-methyl nucleotides, nucleotides containing biotin group, and terminal glyceryl and/or inverted deoxy abasic residue, nucleotide containing sterically hindered molecules, such as fluorescent molecules and the like, 3'-deoxyadenosine (cordycepin), 3'-azido-3'-deoxythymidine (AZT), 2',3'-dideoxyinosine (ddI), 2',3'-dideoxy-3'-thiacytidine (3TC), 2',3'-didehydro-2',3'-dideoxythymidine (d4T), nucleotides containing 3'-azido-3'-deoxythymidine (AZT), 2',3'-dideoxy-3'-thiacytidine (3TC) or 2',3'-didehydro-2',3'-dide-oxythymidine (d4T), a nucleotide having a Northern conformation, 2'-methyl-thio-ethyl, 2'-deoxy-2'-fluoro nucleotides, 2'-deoxy-2'-chloro nucleotides, 2'-azido nucleotides, and 2'-O-methyl nucleotides, 6-membered ring nucleotide analogs including hexitol and altritol nucleotide monomers disclosed in WO 2006/047842, etc., mirror nucleotides (for example L-DNA (L-deoxyriboadenosine-3'-phosphate (mirror dA), L-deoxyribocytidine-3'-phosphate (mirror dC), L-deoxyriboguanosine-3'-phosphate (mirror dG), L-deoxyribothymidine-3'-phosphate (mirror image dT)) and L-RNA (L-riboadenosine-3'-phosphate (mirror rA), L-ribocytidine-3'-phosphate (mirror rC), L-riboguanosine-3'-phosphate (mirror rG), L-ribouracil-3'-phosphate (mirror dU), etc.).

Non-limiting examples of the modified nucleotide are also described in, for example, Gaglione and Messere, Mini Rev Med Chem. 2010; 10 (7): 578-95, Deleavey and Damha, Chem Biol. 2012; 19 (8): 937-54, and Bramsen and Kjems, J. Front Genet. 2012; 3: 154.

The anticancer agent comprised in the combination of the present disclosure includes a drug suppressing the growth, infiltration and/or metastasis of a cancer. The anticancer agent includes an alkylating agent, an antitumor antibiotic, an antimetabolite, a platinum agent, a topoisomerase inhibitor, a microtubule inhibitor, a molecular targeted drug, an endocrine therapeutic drug, and the like. The anticancer agent comprised in the combination of the present disclosure is different from the CUGACUC-containing molecule comprised in the combination of the present disclosure. Thus, the anticancer agent comprised in the combination of the present disclosure does not comprise the nucleotide sequence of SEQ ID NO: 1.

In one embodiment, the anticancer agent comprises a molecular targeted drug that targets a BcL2 family molecule, for example, an inhibitor of a BcL2 family molecule. The inhibitor of a BcL2 family molecule includes, for example, an expression inhibitor of the BcL2 family molecule and a function inhibitor of the BcL2 family molecule. Examples of the expression inhibitor of the BcL2 family molecule include, but are not limited to, inhibitory nucleic acid molecules, such as RNAi molecules, microRNA, microRNA mimic, constructs for genome editing, ribozymes, and antisense nucleic acids, targeting the BcL2 family molecule, and constructs and vectors for the expression thereof. The RNAi (RNA interference) molecule means any molecule having RNAi activity. Examples thereof include, but are not limited to, RNA such as siRNA (small interfering RNA), shRNA (short hairpin RNA), ddRNA (DNA-directed RNA), piRNA (Piwi-interacting RNA), and rasiRNA (repeat associated siRNA), and variants thereof. Examples of the construct for genome editing include, but are not limited to, constructs based on CRISPR-Cas9, TALEN, and ZFN. The construct for genome editing based on CRISPR-Cas9 may comprise guide RNA and a nucleic acid encoding Cas9 in the same construct or in separate constructs.

In a preferred embodiment, the BcL2 family molecule is an antiapoptotic BcL2 family molecule. The antiapoptotic BcL2 family molecule includes BcL-2, BcL-XL, BFL1, BcL-W, and the like. In a particularly preferred embodiment, the BcL2 family molecule is BcL-XL. The antiapoptotic BcL2 family molecule is considered to suppress apoptosis through interaction with a proapoptotic BcL2 family molecule (e.g., multidomain proteins such as BAX, BOK, and BAK, and BH3-only proteins such as BIM, BAD, BID, NOXA, PUMA (Bbc3), BMF, HRK, and BIK).

Examples of the expression inhibitor of the antiapoptotic BcL2 family molecule include, but are not limited to, inhibitory nucleic acid molecules, such as RNAi molecules, microRNA, microRNA mimic, constructs for genome editing, ribozymes, and antisense nucleic acids, targeting the antiapoptotic BcL2 family molecule, for example, a molecule selected from BcL-2, BcL-XL, BFL1 and BcL-W, and constructs and vectors for the expression thereof. Since BcL-XL, BFL1 and BcL-W are encoded by BCL2L1, BCL2A1 and BCL2L2 genes, respectively, the expression inhibitor of BcL-XL, BFL1 or BcL-W may target the BCL2L1, BCL2A1 or BCL2L2 gene.

Examples of the function inhibitor of the antiapoptotic BcL2 family molecule include, but are not limited to, drugs inhibiting the binding of the antiapoptotic BcL2 family molecule to a BH3-only protein. Examples of such a drug include BH3 mimic. Non-limiting examples of the BH3 mimic include ABT-737, ABT-263, ABT-199, WEHI-539, BXI-61, BXI-72, GX15-070, S1, JY-1-106, ApoG2, BI97C1, TW-37, MIM1, MS1 and UMI-77. Non-limiting examples of the drug inhibiting the binding of BcL-XL to a BH3-only protein include ABT-737, ABT-263, WEHI-539, BXI-61, BXI-72, GX15-070, JY-1-106, BI97C1 and TW-37.

The combination of the present disclosure may comprise one or more CUGACUC-containing molecules and anticancer agents. Thus, the combination of the present disclosure can comprise one or more CUGACUC-containing molecules and one or more anticancer agents. In the combination of the present disclosure, the CUGACUC-containing molecule and the anticancer agent may be present as separate molecules or may be present in the same molecule. For example, the combination of the present disclosure may comprise the CUGACUC-containing molecule and the anticancer agent as separate molecules. When the CUGACUC-containing molecule is a nucleic acid molecule, the combination of the present disclosure may be a nucleic acid construct or vector comprising a nucleotide sequence encoding the CUGACUC-containing molecule and a nucleotide sequence encoding the anticancer agent in the same molecule. When the combination of the present disclosure comprises the CUGACUC-containing molecule and the anticancer agent as separate molecules, these molecules may be comprised in the same preparation or may be comprised in separate preparations. Non-limiting examples of the former case include the case where the CUGACUC-containing molecule and the anticancer agent are comprised in the same delivery system.

In case the combination of the present disclosure comprises a nucleic acid molecule, the nucleic acid molecule may be delivered or administered together with any known delivery carrier having an effect of assisting in, promoting or facilitating delivery to a site of action, or may be delivered or administered directly without such a delivery carrier. A viral vector or a non-viral vector can be used as the delivery carrier.

Examples of the viral vector include, but are not limited to, vectors based on adenovirus, adeno-associated virus (AAV), retrovirus, vaccinia virus, poxvirus, lentivirus, and herpes virus. The viral vector may be oncolytic. The oncolytic viral vector is particularly useful for the treatment of a cancer.

Examples of the non-viral vector include, but are not limited to, carriers in a particle form such as polymer particles, lipid particles and inorganic particles, and a bacterial vector. Nanoparticles having a nano level of size can be used as the carrier in a particle form. Examples of the polymer particles include, but are not limited to, those comprising polymers such as cationic polymers, polyamidoamine (PAMAM), chitosan, cyclodextrin, poly(lactic-co-glycolic acid) (PLGA), poly(lactic-co-caprolactonic acid) (PLCA), poly(β amino ester), and atelocollagen. The lipid particles include liposomes, non-liposomal lipid particles, and the like. The liposome is a vesicle having a lumen surrounded by a lipid bilayer, and the non-liposomal lipid particles are lipid particles having no such structure. Examples of the inorganic particles include gold nanoparticles, quantum dots, silica nanoparticles, iron oxide nanoparticles (e.g., superparamagnetic iron oxide nanoparticles (SPION)), nanotubes (e.g., carbon nanotubes (CNT)), nanodiamond, and fullerene. Examples of the bacterial vector include, but are not limited to, vectors based on *Listeria* bacterium, bifidus, and salmonella.

The combination of the present disclosure can be systemically administered or locally administered *ex vivo* or *in vivo* to a tissue concerned via skin application, transdermal application or injection (intravenous injection, intradermal injection, subcutaneous injection, intramuscular injection, intraarterial injection, drip injection, etc.).

The combination of the present disclosure can be delivered through a delivery system suitable for a purpose. The delivery system may include, for example, aqueous and non-aqueous gels, creams, double emulsions, microemulsions, liposomes, ointments, aqueous and non-aqueous solutions, lotions, aerosols, hydrocarbon bases and powders and can comprise excipients, for example, solubilizers, penetration enhancers (e.g., fatty acids, fatty acid esters, aliphatic alcohols and amino acids) and hydrophilic polymers (e.g., polycarbophil and polyvinylpyrrolidone). In one embodiment, the pharmaceutically acceptable carrier is a liposome or a transdermal delivery enhancer.

The delivery system may include patches, tablets, suppositories, pessaries, gels and creams and can comprise excipients, for example, solubilizers and enhancers (e.g., propylene glycol, bile salt and amino acids) and other vehicles (e.g., polyethylene glycol, fatty acid esters and derivatives, and hydrophilic polymers, for example, hydroxypropylmethylcellulose and hyaluronic acid).

Approaches and systems useful in the delivery of the combination of the present disclosure are described in, for example, Rettig and Behlke, Mol Ther. 2012; 20 (3): 483-512, Kraft et al., J Pharm Sci. 2014; 103 (1): 29-52, Hong and Nam, Theranostics. 2014; 4 (12): 1211-32, and Kaczmarek et al., Genome Med. 2017; 9 (1): 60.

In some embodiments, the present disclosure relates to a composition comprising the combination of the present disclosure (hereinafter, also referred to as the composition of the present disclosure). The composition of the present disclosure may comprise any carrier, diluent, delivery vehicle, delivery system, and the like, mentioned above, in addition to the composition of the present disclosure. The composition of the present disclosure can be used in the treatment of a disease such as a cancer. Thus, the composition of the present disclosure may serve as a pharmaceutical composition for the treatment of a disease such as a cancer (hereinafter, also referred to as the pharmaceutical composition of the present disclosure). The pharmaceutical composition of the present disclosure may comprise one or more pharmaceutically acceptable additives (e.g., surfactants, carriers, diluents, and excipients). The pharmaceutically acceptable additives are well known in the medical field and described in, for example, Remington's Pharmaceutical Sciences, 18th Ed., Mack Publishing Co., Easton, PA (1990), which is incorporated herein by reference in its entirety.

In some embodiments, the combination and the pharmaceutical composition of the present disclosure can be used for the treatment of a cancer. The cancer includes epithelial malignant tumor and non-epithelial malignant tumor. The cancer to be treated includes, but not limited to, for example, brain tumor, head and neck cancer, breast cancer, lung cancer, oral cancer, esophageal cancer, stomach cancer, duodenal cancer, appendix cancer, colon cancer, rectal cancer, liver cancer, pancreatic cancer, gallbladder cancer, bile duct cancer, anal cancer, kidney cancer, ureteral cancer, bladder cancer, prostate cancer, penile cancer, testicular cancer, uterine cancer, cervical cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and leukemia. The cancer may be present in any site, for example, the brain, the head and the neck, the chest, extremities, the lung, the heart, thymus gland, the esophagus, the stomach, the small intestine (the duodenum, the jejunum, and the ileum), the large intestine (the colon, the cecum, the appendix, and the rectum), the liver, the pancreas, the gallbladder, the anus, the kidney, the ureter, the bladder, the prostate, the penis, the testis, the uterus, the ovary, the vulva, the vagina, the skin, striated muscle, smooth muscle, synovium, cartilage, bone, thyroid gland, adrenal gland, peritoneum, mesentery, bone marrow, blood, the vascular system, the lymphatic system such as lymph nodes, and lymph.

In a particular embodiment, the combination and the pharmaceutical composition of the present disclosure can be used for the treatment of a cancer expressing BcL-XL. In a preferred embodiment, BcL-XL in the cancer is overexpressed. Whether or not a certain cancer expresses BcL-XL or whether or not a certain cancer overexpresses BcL-XL is known from a literature or the like or can be determined, for example, by detecting the expression of BcL-XL in cancer cells constituting the cancer. The expression of BcL-XL can be determined by a known detection approach, for example, by detecting a nucleic acid molecule encoding BcL-XL (Bcl2L1) via various hybridization methods, Northern blotting, Southern blotting, or various PCR methods using a nucleic acid specifically hybridizing to the nucleic acid molecule or a unique fragment thereof, or by detecting BcL-XL via a known protein detection approach, for example, an immunoprecipitation method using an antibody, EIA (e.g., ELISA), RIA (e.g., IRMA, RAST, and RIST), Western blotting, an immunohistochemical method, an immunocytochemical method, or flow cytometry, without limitations. Since the overexpression of BcL-XL in cancer cells may be caused by the amplification of Bcl2L1 gene, the amplification of the Bcl2L1 gene can be used as an index for the overexpression of BcL-XL. It has been reported that the amplification of the Bcl2L1 gene is found in bladder cancer, breast cancer, head and neck cancer, lung cancer, stomach cancer, uterus cancer, and the like (Campbell and Tait, Open Biol. 2018; 8 (5): 180002).

The cancer expressing BcL-XL may be resistant to the suppression of BcL-XL. The suppression of BcL-XL includes the suppression of BcL-XL expression, the suppression of a BcL-XL function, and the like. The resistance of a certain cancer to the suppression of BcL-XL is known from a literature or the like or can be determined, for example, by evaluating the effect of a BcL-XL-suppressing agent on cancer tissues or cancer cells thereof by the action of the agent. For example, when the effect of the BcL-XL-suppressing agent on the cancer tissues or the cancer cells to be evaluated is smaller than that on tissues or cells sensitive to the BcL-XL-suppressing agent (positive control) or is equivalent to or more than that on tissues or cells resistant to the BcL-XL-suppressing agent (negative control), the cancer tissues or the cancer cells to be evaluated can be determined as being resistant to the suppression of BcL-XL. Examples of the cell line sensitive to the BcL-XL-suppressing agent include, but are not limited to, H358, H838, H1975, AsPC-1, and BxPC-3. Examples of the cell line resistant to the BcL-XL-suppressing agent include, but are not limited to, A549, MCF7, BT-20, PANC-1, MIA PaCa-2, KP-3, KP-4, HCT116, SUIT-2, SW1990, SAOS-2, HeLa, HepG2, and CFPAC-1. Examples of the BcL-XL-suppressing agent include: BcL-XL expression-suppressing agents including inhibitory nucleic acid molecules, such as RNAi molecules, microRNA, microRNA mimic, constructs for genome editing, ribozymes, and antisense nucleic acids, targeting BcL-XL, and constructs and vectors for the expression thereof; and BcL-XL function-suppressing agents including BH3 mimic. Examples of the effect of the BcL-XL-suppressing agent include the suppression of cell proliferation and the induction of apoptosis. The suppression of cell proliferation or the induction of apoptosis can be evaluated by use of various known approaches.

In a particular embodiment, the combination and the pharmaceutical composition of the present disclosure can be used for the treatment of a cancer expressing a gene whose expression can be suppressed by the CUGACUC-containing molecule. The gene whose expression can be suppressed by the CUGACUC-containing molecule is as described above with respect to the combination of the present disclosure. The cancer expressing the gene whose expression can be suppressed by the CUGACUC-containing molecule may be resistant to treatment with the CUGACUC-containing molecule. The resistance of a certain cancer to the treatment with the CUGACUC-containing molecule is known from a literature or the like or can be determined, for example, by evaluating the effect of the CUGACUC-containing molecule on cancer tissues or cancer cells thereof by the action of the molecule. For example, when the effect of the CUGACUC-containing molecule on the cancer tissues or the cancer cells to be evaluated is smaller than that on tissues or cells known to be sensitive to the CUGACUC-containing molecule (positive control) or is equivalent to or more than that on tissues or cells known to be resistant to the CUGACUC-containing molecule (negative control), the cancer tissues or the cancer cells to be evaluated can be determined as being resistant to the treatment with the CUGACUC-containing molecule. Examples of cell lines sensitive to the treatment with the CUGACUC-containing molecule include, but are not limited to, A549, MIA and PaCa-2, and examples of cell lines resistant to the treatment with the CUGACUC-containing molecule include, but are not limited to, HCT116, PANC-1, CFPAC-1, SUIT-2, and SW1990. Examples of the CUGACUC-containing molecule are as described above with respect to the combination of the present disclosure. Examples of the effect of the CUGACUC-containing molecule include the suppression of cell proliferation and the induction of apoptosis. The suppression of cell proliferation or the induction of apoptosis can be evaluated by use of various known approaches.

In a particularly preferred embodiment, the combination and the pharmaceutical composition of the present disclosure can be used for the treatment of a cancer expressing both genes whose expression can be suppressed by BcL-XL and the CUGACUC-containing molecule, respectively. Such a cancer may be resistant to the suppression of BcL-XL and resistant to treatment with the CUGACUC-containing molecule. The expression of BcL-XL, the expression of the gene whose expression can be suppressed by the CUGACUC-containing molecule, the resistance to the suppression of BcL-XL and the resistance to the treatment with the CUGACUC-containing molecule are as described above. Examples of cell lines resistant to the suppression of BcL-XL and resistant to the treatment with the CUGACUC-containing molecule include, but are not limited to, HCT116, CFPAC-1, SUIT-2, and SW1990.

The combination and the pharmaceutical composition of the present disclosure can also be used for the treatment of a disease caused by abnormality in apoptosis, for example, a disease caused by the abnormal proliferation of cells. Examples of the disease caused by the abnormal proliferation of cells include, but are not limited to, benign or malignant tumor, hyperplasia, keloid, Cushing's syndrome, primary aldosteronism, erythroplakia, polycythemia rubra vera, leukoplakia, hyperplastic scar, lichen planus and lentiginosis.

The combination and the pharmaceutical composition of the present disclosure can also be used for the treatment of a disease caused by the expression of BcL-XL, for example, a disease caused by the abnormal proliferation of cells associated with the expression of BcL-XL. Examples of the disease caused by the abnormal proliferation of cells include, but are not limited to, benign or malignant tumor and lymphoproliferative diseases.

In the present disclosure, the "treatment" includes every type of medically acceptable prophylactic and/or therapeutic intervention aimed at cure, transient remission or prevention, etc. of a disease. The "treatment" includes medically acceptable intervention for various purposes including, for example, the delay or arrest of progression of a disease, the regression or disappearance of a lesion, the prevention of onset of the disease or the prevention of recurrence of the disease. Thus, the combination and the pharmaceutical composition can be used in the treatment and/or the prevention of a disease.

The combination or the pharmaceutical composition of the present disclosure may be administered through various routes including both oral and parenteral routes, for example, but not limited to, oral, buccal, mouth, intravenous, intramuscular, subcutaneous, intradermal, local, rectal, intraarterial, intraportal, intraventricular, transmucosal, transdermal, intranasal, intraperitoneal, intratracheal, intrapulmonary and intrauterine routes, and may be formulated into a dosage form suitable for each administration route. Any ones known in the art can be appropriately adopted to such a dosage form and a formulation method (see e.g., Remington's Pharmaceutical Sciences, 18th Ed., Mack Publishing Co., Easton, PA (1990)).

Examples of the dosage form suitable for oral administration include, but are not limited to, powders, granules, tablets, capsules, solutions, suspensions, emulsions, gels, and syrups. Examples of the dosage form suitable for parenteral administration include injections such as solution-type injections, suspension-type injections, emulsion-type injection, and injections to be prepared at the time of use. The preparation for parenteral administration can be in the form of an aqueous or non-aqueous isotonic sterile solution or suspension.

The composition according to the present disclosure may be supplied in any form and may be provided in a form capable of being prepared at the time of use, for example, a form capable of being prepared by a physician and/or a pharmacist, a nurse, or other paramedical staff, etc. in or near medical setting, from the viewpoint of preservation stability. In this case, the composition is provided in one or more containers comprising at least one component essential therefor, and prepared before use, for example, within 24 hours before use, preferably within 3 hours before use, more preferably immediately before use. For the preparation, a reagent, a solvent, pharmacy equipment, and the like usually available in a place of preparation can be appropriately used.

In further aspects, the present disclosure relates to a kit or a pack for preparing the combination or the composition and/or for treating a disease, comprising the combination or the composition according to the present disclosure or a component thereof, and the combination or the composition, or a necessary component thereof that is provided in the form of such a kit or a pack. Each component of the combination or the composition comprised in this kit or pack is as described above with respect to the combination or the composition. The present kit may further comprise instructions as to a method for preparing or using (e.g., administering) the combination or the composition, for example, instruction manuals, and a medium, for example, a flexible disc, CD, DVD, a Blu-ray disc, a memory card, or a USB memory, in which information on the use method is recorded, in addition to those described above. Also, the kit or the pack may comprise all components for completing the combination or the composition or may not necessarily comprise all the components. Thus, the kit or the pack may not comprise a reagent or a solvent usually available in a medical setting, an experimental facility, etc., for example, sterile water, saline, or a glucose solution.

In an alternative aspect, the present disclosure relates to a method for treating a cancer, a disease caused by abnormal apoptosis or a disease caused by the expression of BcL-XL, the method comprising the step of administering an effective amount of the combination or the pharmaceutical composition according to the present disclosure to a subject in need thereof (hereinafter, also referred to as the "treatment method of the present disclosure"). In this context, the effective amount is, for example, an amount in which the onset and recurrence of the disease are prevented, or the disease is cured.

In the treatment method, the specific dose of the combination or the pharmaceutical composition to be administered to the subject may be determined in consideration of various conditions as to the subject in need of the administration, for example, the type of the target, the purpose of the method, a therapeutic regimen, the type of the disease, the severity of symptoms, the general health state, age, and body weight of the subject, the sex of the subject, diets, the timing and frequency of administration, a concurrent drug, responsiveness to therapy, and compliance with therapy. The total daily dose of the combination or the pharmaceutical composition is not limited and may be, for example, about 1 µg/kg to about 1000 mg/kg body weight, about 10 µg/kg to about 100 mg/kg body weight, or about 100 µg/kg to about 10 mg/kg body weight, in terms of the amount of the combination. Alternatively, the dose may be calculated on the basis of the surface area of a patient.

The administration route includes various routes including both oral and parenteral routes, for example, oral, buccal, mouth, intravenous, intramuscular, subcutaneous, intradermal, local, rectal, intraarterial, intraportal, intraventricular, transmucosal, transdermal, intranasal, intraperitoneal, intratracheal, intrapulmonary and intrauterine routes.

The frequency of administration differs depending on the properties of the preparation or the composition used or the conditions of the subject as described above and may be, for example, plural times per day (i.e., 2, 3, 4 or 5 or more times per day), once a day, every few days (i.e., every 2, 3, 4, 5, 6, or 7 days), several times a week (e.g., 2, 3, or 4 times a week), every week, or every few weeks (i.e., every 2, 3, or 4 weeks).

In the present disclosure, the term "subject" means any individual organism, preferably animal, more preferably mammalian, further preferably human individual. The subject may be healthy (e.g., have no particular or any disease) or may be affected by some disease. When the treatment of a disease associated with a target nucleic acid molecule is intended, for example, the subject typically means a subject affected by the disease or having a risk of being affected by the disease.

In another aspect, the present disclosure relates to a molecule comprising the nucleotide sequence of SEQ ID NO: 1 for treating a cancer, a disease caused by abnormality in apoptosis and/or a disease caused by the expression of BcL-XL in combination with an anticancer agent (hereinafter, also referred to as the "CUGACUC sequence-containing molecule A of the present disclosure" or the "CUGACUC-containing molecule A of the present disclosure").

Each of the terms "anticancer agent", "cancer", "disease caused by abnormality in apoptosis", "disease caused by the expression of BcL-XL", "treatment", and "molecule comprising the nucleotide sequence of SEQ ID NO: 1" for the CUGACUC-containing molecule A of the present disclosure is as described above with respect to the CUGACUC-containing molecule of the present disclosure. The CUGACUC-containing molecule A of the present disclosure can be provided together with instructions (e.g., a package insert) as to the treatment of a cancer, a disease caused by abnormality in apoptosis and/or a disease caused by the expression of BcL-XL in combination with an anticancer agent.

In an alternative aspect, the present disclosure relates to a pharmaceutical composition for treating a cancer, a disease caused by abnormality in apoptosis and/or a disease caused by the expression of BcL-XL in combination with an anticancer agent, the pharmaceutical composition comprising a molecule comprising the nucleotide sequence of SEQ ID NO: 1 (hereinafter, also referred to as the "pharmaceutical composition A of the present disclosure".

Each of the terms "molecule comprising the nucleotide sequence of SEQ ID NO: 1", "anticancer agent", "cancer", "disease caused by abnormality in apoptosis", "disease caused by the expression of BcL-XL", and "treatment" for the pharmaceutical composition A of the present disclosure is as described above with respect to the CUGACUC-containing molecule or the pharmaceutical composition of the present disclosure. The pharmaceutical composition A of the present disclosure does not have to comprise the anticancer agent. The pharmaceutical composition A of the present disclosure can be provided together with instructions (e.g., a package insert) as to the treatment of a cancer, a disease caused by abnormality in apoptosis and/or a disease caused by the expression of BcL-XL in combination with an anticancer agent.

In an alternative aspect, the present disclosure relates to use of (1) a molecule comprising the nucleotide sequence of SEQ ID NO: 1 and (2) an anticancer agent in the production of a medicament for the treatment of a cancer, a disease caused by abnormality in apoptosis and/or a disease caused by the expression of BcL-XL (hereinafter, also referred to as the "use of present disclosure").

Each of the terms "cancer", "disease caused by abnormality in apoptosis", "disease caused by the expression of BcL-XL", "treatment", "molecule comprising the nucleotide sequence of SEQ ID NO: 1", and "anticancer agent" for the use of the present disclosure is as described above with respect to the CUGACUC-containing molecule of the present disclosure. The medicament in the use of the present disclosure may comprise the CUGACUC-containing molecule and the anticancer agent in the same preparation or in separate preparations.

In an alternative aspect, the present disclosure relates to use of a molecule comprising the nucleotide sequence of SEQ ID NO: 1 in the production of a medicament for treating a cancer, a disease caused by abnormality in apoptosis and/or a disease caused by the expression of BcL-XL in combination with an anticancer agent (hereinafter, also referred to as the "use A of present disclosure").

Each of the terms "cancer", "disease caused by abnormality in apoptosis", "disease caused by the expression of BcL-XL", "treatment", "molecule comprising the nucleotide sequence of SEQ ID NO: 1", and "anticancer agent" for the use A of the present disclosure is as described above with respect to the CUGACUC-containing molecule or the use of the present disclosure. The medicament in the use A of the present disclosure does not have to comprise the anticancer agent. The medicament can be provided together with instructions (e.g., a package insert) as to the treatment of a cancer, a disease caused by abnormality in apoptosis and/or a disease caused by the expression of BcL-XL in combination with an anticancer agent.

### EXAMPLES

Some embodiments of the present disclosure will be described in more detail with reference to the examples given below. However, these examples are given for illustrative purposes and do not limit the scopes of the embodiments.

### Example 1 Enhancement in anticancer activity by concurrent use of BcL-XL inhibitor and miR-345-related molecule

A BcL-XL inhibitor and/or a miR-345-related molecule were allowed to act on cancer cells and tested for the presence or absence of the effect of concurrent use by using the ability to suppress cell proliferation and the ability to induce apoptosis as indexes.

The BcL-XL inhibitor used was siRNA consisting of the following nucleotide sequences (Compound A). In the sequences, the upper-case letters represent RNA, and the lower-case letters represent DNA.

### Compound A

Sense strand: 5' -GGUAUUGGUGAGUCGGAUCtt-3' (SEQ ID NO: 4)
Antisense strand: 5' -GAUCCGACUCACCAAUACCtt-3' (SEQ ID NO: 5)

As miR-345-related molecule, hsa-miR-345-5p mirVana™ miRNA mimic (ID: MC12733, Ambion, Compound B) was used.

The cancer cells used were of a K-Ras mutation-positive colon cancer cell line HCT116. The cells were cultured in DMEM medium containing 10% inactivated fetal bovine serum (FBS), 100 U/mL penicillin, and 100 µg/mL streptomycin under conditions of 37°C and 5% CO₂.

Transfection with Compound A and/or Compound B was performed as follows: on the day before transfection, the HCT116 cells were seeded to a 6-well tissue culture plastic dish at 0.1 × 10⁵ cells/well for cell number counting experiments and 0.2 × 10⁵ cells/well for Western blot. 25 pmol (25 + 25 pmol for concurrent use) of Compound A and/or Compound B was added into 250 µL of Opti-MEM I Reduced Serum Medium (Invitrogen Corp.) and gently mixed. Next, 5 µL of Lipofectamine RNAiMAX (Invitrogen Corp.) was diluted into 250 µL of Opti-MEM I Reduced Serum Medium and gently mixed. The Compound A and/or Compound B dilution and the Lipofectamine RNAiMAX dilution were combined, gently mixed, and then incubated at room temperature for 15 minutes. During this period, the medium was replaced with 2 mL of Opti-MEM I Reduced Serum Medium. After the incubation for 15 minutes, the complex of Compound A and/or Compound B was added to the cells and incubated at 37°C in the atmosphere containing 5% CO₂. After the incubation for 5 hours, the medium was replaced with 3 mL of DMEM medium containing 10% FBS. For a control experiment, similar operation was performed using Allstars negative control siRNA (Qiagen N.V., Compound C). On day 3 after the transfection, cell extracts were prepared, and the knockdown of BcL-XL as well as change in the expression of Smad1, which is a target molecules of hsa-miR-345, and of activated caspase-3 and PARP which are apoptosis signals induced by the suppression of Bcl-2, was analyzed by Western blot. On day 5 thereafter, the cell number was counted.

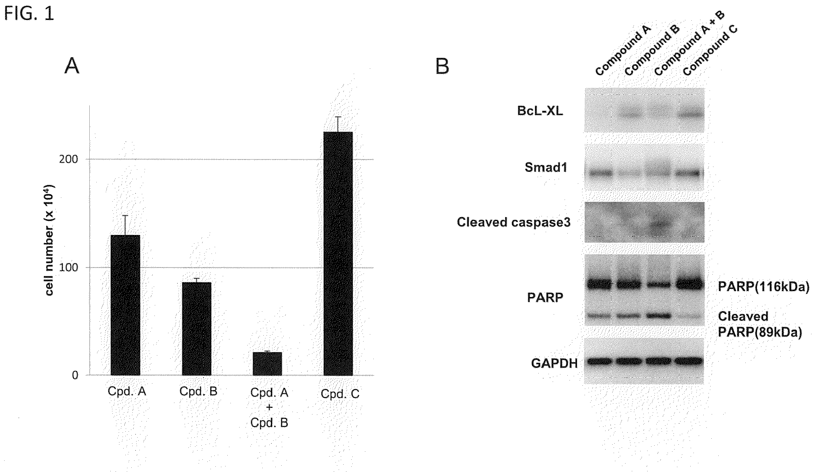

Western blot was performed as follows: the cells were washed with ice-cooled PBS and then lysed by the addition of TNE lysis buffer (1% NP-40, 50 mM Tris-HCl, 150 mM NaCl, 1 mM EDTA, complete Mini EDTA-free (F. Hoffmann-La Roche, Ltd.), and PhosSTOP (F. Hoffmann-La Roche, Ltd.), pH 7.5) and incubation for 30 minutes under ice cooling. Then, the cells were centrifuged for 15 minutes under conditions of 15000 rpm and 4°C, and the supernatant was used as cell extracts. Proteins in the obtained cell extracts were quantified using Micro BCA Protein Assay Kit (Thermo Fisher Scientific Inc.). Red Loading Buffer Pack (New England Biolabs Inc.) was added to 10 µg of the cell extracts, which were then heat-treated (100°C, 5 min) for denaturation. The proteins were separated by SDS-PAGE using SuperSep™ Ace (Wako Pure Chemical Industries, Ltd.). The proteins thus separated were transferred to PVDF transfer membrane (Immobilon-P; Merck Millipore) using a semidry blotting apparatus (Bio-Rad Laboratories, Inc.). The membrane was blocked by incubation at room temperature for 1 hour in PBS supplemented with 5% skimmed milk/0.05% Tween 20 (hereinafter, abbreviated to PBS-T). Subsequently, the membrane was incubated at 4°C for 16 hours with various primary antibodies (Bcl-xL (54H6) Rabbit mAb #2764 (Cell Signaling Technology, Inc. (CST)), PARP Antibody #9542 (CST), Smad1 (D59D7) XP® Rabbit mAb #6944 (CST), Cleaved Caspase-3 (Asp175) (5A1E) Rabbit mAb #9664 (CST), and Anti-GAPDH antibody [6C5] (Abcam plc)) diluted with PBS-T. The membrane was washed with PBS-T and then incubated at room temperature for 60 minutes with corresponding HRP-conjugated anti-mouse or anti-rabbit IgG (CST). The membrane was washed with PBS-T and then reacted with SuperSignal™ West Femto Maximum Sensitivity Substrate (Thermo Fisher Scientific Inc.), followed by chemiluminescence detection using chemidoc (Bio-Rad Laboratories, Inc.). In the washing between the operations, shaking for 5 minutes was performed three times using PBS-T. The results are shown in Figure 1. As shown in Figure 1, stronger suppression of proliferation was found in the concurrent use of Compound A and Compound B than in Compound A alone and Compound B alone. Furthermore, stronger expression of cleaved caspase-3 and cleaved PARP was found in the concurrent use of Compound A and Compound B than in Compound A alone and Compound B alone, suggesting that the concurrent use induced apoptosis more strongly than each compound alone did.

### Example 2 Testing of anticancer activity by combination of suppression of BcL-XL expression by BcL-XL inhibitor with administration of miR-345-related molecule

The anticancer activity of a miR-345-related molecule was evaluated in cancer cells in which the expression of BcL-XL was suppressed by a BcL-XL inhibitor.

The BcL-XL inhibitor used was pCG SapI vector (all-in-one vector using the CRISPR/Cas9 system; Takei et al., Sci Rep. 2017; 7 (1): 9389) having an insert of a BcL-XL-specific gRNA sequence (gtttgaactgcggtaccggcggg, SEQ ID NO: 6) (Compound D).

The miR-345-related molecule used was Compound B used in Example 1.

A cell line in which the expression of BcL-XL was suppressed (BcL-XL-knockout cell line (KO)) was established by introducing Compound D to BcL-XL wild-type HCT116 cells using Lipofectamine 3000 (Invitrogen Corp.), and picking up a single clone by drug selection.

In the experiment, a clone was used which was confirmed to have deficiency in BcL-XL gene locus and the disappearance of BcL-XL protein expression by genotyping PCR and Western blotting. A BcL-XL wild-type HCT116 cell line (WT) as a control was established by introducing pCG SapI vector (Compound E) having no insert sequence thereto using Lipofectamine 3000 (Invitrogen Corp.), and picking up a single clone by drug selection.

Transfection with Compound B was performed as follows: on the day before transfection, the BcL-XL-knockout or wild-type HCT116 cells were seeded at 0.1 × 10⁵ cells/well to a 6-well tissue culture plastic dish. 25 pmol of Compound B was added into 250 µL of Opti-MEM I Reduced Serum Medium (Invitrogen Corp.) and gently mixed. Next, 5 µL of Lipofectamine RNAiMAX (Invitrogen Corp.) was diluted into 250 µL of Opti-MEM I Reduced Serum Medium and gently mixed. The Compound B dilution and the Lipofectamine RNAiMAX dilution were combined, gently mixed, and then incubated at room temperature for 15 minutes. During this period, the medium was replaced with 2 mL of Opti-MEM I Reduced Serum Medium. After the incubation for 15 minutes, the complex of Compound B with Lipofectamine RNAiMAX was added to the cells and incubated at 37°C in the atmosphere containing 5% CO₂. After the incubation for 5 hours, the medium was replaced with 3 mL of DMEM medium containing 10% FBS. For a control experiment, similar operation was performed using Compound C (Qiagen N.V.). On day 5 after the transfection, the cell number was counted. As shown in results in Figure 2, the proliferation-suppressing effect of Compound B appeared more strongly in the BcL-XL-knockout cells than in the BcL-XL wild-type cells. These results indicate the further evidence that the concurrent use of the BcL-XL inhibitor and the miR-345-related molecule enhances anticancer activity more than the miR-345-related molecule alone does. It is also evident that even if the BcL-XL inhibitor and the miR-345-related molecule are allowed to act at different timings, the effect brought about by the concurrent use can be obtained as long as the expression of BcL-XL is suppressed.

### Example 3 Testing of anticancer activity by combination of overexpression of miR-345 by miR-345-related molecule with administration of BcL-XL inhibitor

The anticancer activity of a BcL-XL inhibitor was evaluated in cancer cells in which miR-345 was overexpressed by a miR-345-related molecule.

The BcL-XL inhibitor used was Compound A used in Example 1.

The miR-345-related molecule used was Expression plasmid for human microRNA MIR345 (MI0000825, OriGene Technologies, Inc., Compound F).

A cell line overexpressing miR-345 was established by introducing Compound E to hsa-miR-345 wild-type HCT116 cells using Lipofectamine 3000 (Invitrogen Corp.), and picking up a single clone by drug selection. In the experiment, a clone was used which was confirmed to have the increased expression of hsa-miR-345-5p by qPCR. A hsa-miR-345 wild-type HCT116 cell line as a control was established by introducing Expression plasmid pCMV-MIR Vector (PCMVMIR, OriGene Technologies, Inc., Compound G) to hsa-miR-345 wild-type HCT116 cells using Lipofectamine 3000 (Invitrogen Corp.), and picking up a single clone by drug selection.

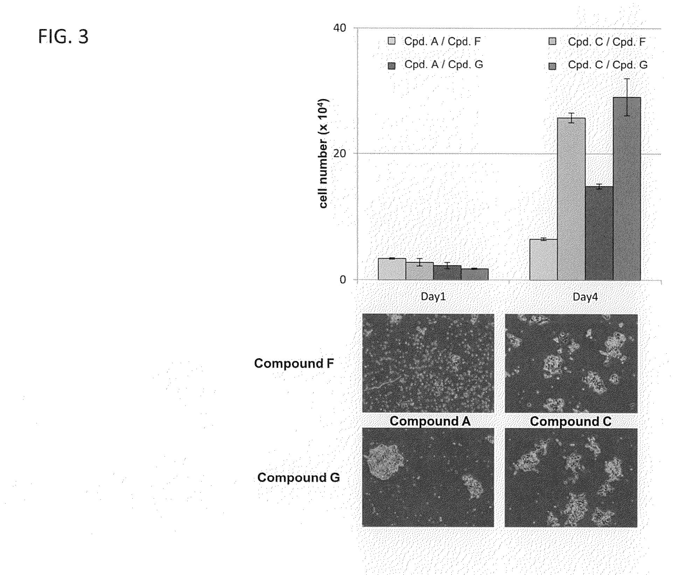

Transfection with Compound A was performed as follows: on the day before transfection, the hsa-miR-345-overexpressing HCT116 cells or the control HCT116 cells were seeded at 0.1 × 10⁵ cells/well to a 6-well tissue culture plastic dish. 25 pmol of Compound A was added into 250 µL of Opti-MEM I Reduced Serum Medium (Invitrogen Corp.) and gently mixed. Next, 5 µL of Lipofectamine RNAiMAX (Invitrogen Corp.) was diluted into 250 µL of Opti-MEM I Reduced Serum Medium and gently mixed. The Compound A dilution and the Lipofectamine RNAiMAX dilution were combined, gently mixed, and then incubated at room temperature for 15 minutes. During this period, the medium was replaced with 2 mL of Opti-MEM I Reduced Serum Medium. After the incubation for 15 minutes, the complex of Compound A with Lipofectamine RNAiMAX was added to the cells and incubated at 37°C in the atmosphere containing 5% CO₂. After the incubation for 5 hours, the medium was replaced with 3 mL of DMEM medium containing 10% FBS. For a control experiment, similar operation was performed using Allstars negative control siRNA (Qiagen N.V., Compound C). On days 1 and 4 after the transfection, the cell number was counted. As shown in results in Figure 3, the cell proliferation-suppressing effect of Compound A appeared more strongly in the hsa-miR-345-overexpressing line than in the hsa-miR-345 wild-type line. These results indicate the further evidence that the concurrent use of the BcL-XL inhibitor and the miR-345-related molecule enhances anticancer activity more than the BcL-XL inhibitor alone does. It is also evident that even if the BcL-XL inhibitor and the miR-345-related molecule are allowed to act at different timings, the effect brought about by the concurrent use can be obtained as long as the expression of miR-345 is enhanced.

### Example 4 Testing of anticancer activity by combination of suppression of BcL-XL expression by BcL-XL inhibitor with administration of molecule comprising no CUGACUC sequence

The anticancer activity of microRNA mimic comprising no CUGACUC sequence was evaluated in cancer cells in which the expression of BcL-XL was suppressed by a BcL-XL inhibitor.

The BcL-XL inhibitor used was Compound D used in Example 2.

The molecule comprising no CUGACUC sequence used was hsa-miR-1182 MISSION® miRNA mimic (ID: HMI0051, Sigma-Aldrich Co. LLC, Compound H).

Compound B was used as a positive control, and mirVana™ miRNA mimic Negative Control #1 (Ambion, Inc., Compound I) was used as a negative control.

A cell line in which the expression of BcL-XL was suppressed (BcL-XL-knockout cell line (KO)) and a BcL-XL wild-type HCT116 cell line (WT) for a control were prepared in the same way as in Example 2.

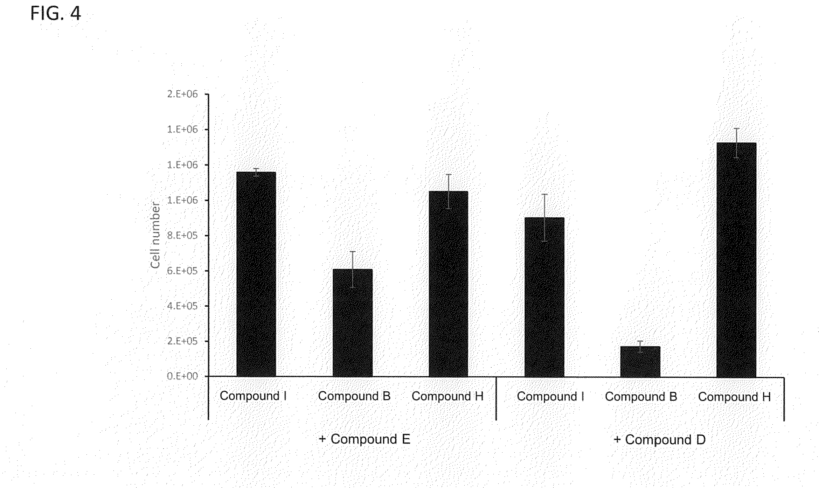

Transfection with Compound B, H or I was performed as follows: on the day before transfection, the BcL-XL-knockout or wild-type HCT116 cells were seeded at 0.25 × 10⁵ cells/well to a 6-well tissue culture plastic dish. 25 pmol of Compound B, H or I was added into 250 µL of Opti-MEM I Reduced Serum Medium (Invitrogen Corp.) and gently mixed. Next, 5 µL of Lipofectamine RNAiMAX (Invitrogen Corp.) was diluted into 250 µL of Opti-MEM I Reduced Serum Medium and gently mixed. The Compound B, H or I dilution and the Lipofectamine RNAiMAX dilution were combined, gently mixed, and then incubated at room temperature for 15 minutes. During this period, the medium was replaced with 2 mL of Opti-MEM I Reduced Serum Medium. After the incubation for 15 minutes, the complex of Compound B, H or I with Lipofectamine RNAiMAX was added to the cells and incubated at 37°C in the atmosphere containing 5% CO₂. After the incubation for 5 hours, the medium was replaced with 3 mL of DMEM medium containing 10% FBS. For a control experiment, similar operation was performed using Allstars negative control siRNA (Qiagen N.V., Compound C). On day 6 after the transfection, the cell number was counted. As shown in results in Figure 4, only Compound B comprising the CUGACUC sequence exhibited a stronger proliferation-suppressing effect in the BcL-XL-knockout cells than in the BcL-XL wild-type cells. This indicates that the CUGACUC sequence is necessary for enhancement in anticancer activity by the concurrent use with the BcL-XL inhibitor.

### Example 5 Influence of combination of BcL-XL inhibitor and miR-345-related molecule on normal cell

A combination of a BcL-XL inhibitor and a miR-345-related molecule was tested for the presence or absence of its influence on the proliferation of normal cells.

The BcL-XL inhibitor used was Compound A, and the miR-345-related molecule used was Compound B. Compound C was used as a negative control of the BcL-XL inhibitor, and Compound I was used as a negative control of the miR-345-related molecule.

The normal cells used were human normal dermal fibroblasts NHDF-Ad-Der Fibroblasts (Lonza Group AG, #CC-2511). The cells were cultured in Fibroblast Basal Medium (FBM™, Lonza Group AG, #CC-3131) containing FGM™-2 SingleQuots™ (Lonza Group AG, #CC-4126) under conditions of 37°C and 5% CO₂.

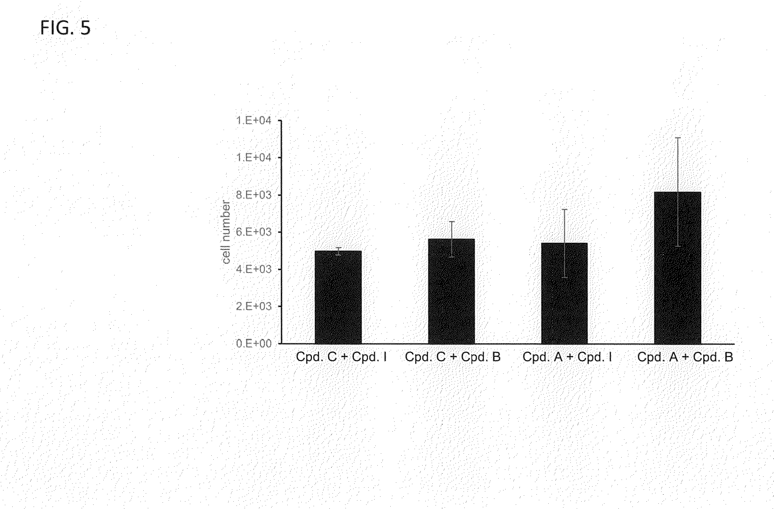

Transfection with the BcL-XL inhibitor and/or the miR-345-related molecule was performed as follows: on the day before transfection, NHDF was seeded at 0.15 × 10⁴ cells/well to a 96-well tissue culture plastic dish. 1.1 + 1.1 pmol of Compound A and Compound I, Compound B and Compound C, or Compound A and Compound B was added into 5 µL of Opti-MEM I Reduced Serum Medium (Invitrogen Corp.) and gently mixed. Next, 0.12 µL of Lipofectamine RNAiMAX (Invitrogen Corp.) was diluted into 4.88 µL of Opti-MEM I Reduced Serum Medium and gently mixed. Each compound dilution and the Lipofectamine RNAiMAX dilution were combined, gently mixed, and then incubated at room temperature for 15 minutes. During this period, the medium was replaced with 100 µL of Opti-MEM I Reduced Serum Medium. After the incubation for 15 minutes, the complex of each compound with Lipofectamine RNAiMAX was added to the cells and incubated at 37°C in the atmosphere containing 5% CO₂. After the incubation for 5 hours, the medium was replaced with 100 µL of DMEM medium containing 10% FBS. For a control experiment, similar operation was performed using Compound C and Compound I. On day 4 after the transfection, Hoechst 33342 (Thermo Fisher Scientific Inc., #H3570) and propidium iodide (Wako Pure Chemical Industries, Ltd., #169-26281) were added at final concentrations of 5 µg/mL and 2 µg/mL, respectively, to the medium. Live and dead cell numbers were counted using Celigo® Image Cytometer (Nexcelom Bioscience, LLC, #Celigo-106-0448) in which the number of cells stained with Hoechst 33342 was regarded as the total of the live and dead cell numbers and the number of cells stained with propidium iodide was regarded as the dead cell number. As shown in results in Figure 5, neither the BcL-XL inhibitor alone or the miR-345-related molecule alone nor their concurrent use suppressed the proliferation of normal cells, as compared with the control.

Those skilled in the art will understand that many various modifications can be made in the present invention without departing from the spirit of the present invention. Thus, it should be understood that the modes of the present invention described in the present specification are given merely for illustrative purposes and are not intended to limit the scope of the present invention.

## Claims

1. A combination comprising (1) a molecule comprising the nucleotide sequence of SEQ ID NO: 1 and (2) an anticancer agent.

2. The combination according to claim 1, wherein the anticancer agent is an inhibitor of a BcL2 family molecule.

3. The combination according to claim 2, wherein the inhibitor of a BcL2 family molecule is an expression inhibitor of the BcL2 family molecule or a function inhibitor of the BcL2 family molecule.

4. The combination according to claim 3, wherein the BcL2 family molecule is BcL-XL.

5. The combination according to any one of claims 1 to 4, wherein the molecule (1) suppresses the expression of a gene comprising a nucleotide sequence having complementarity to the nucleotide sequence of SEQ ID NO: 1.

6. The combination according to any one of claims 1 to 5, wherein the molecule (1) is a miR-345-related molecule selected from double-stranded mature microRNA, pre-microRNA, pri-microRNA, and microRNA mimic of miR-345, and a vector for the expression of one or more thereof.

7. A pharmaceutical composition comprising a combination according to any one of claims 1 to 6 and a pharmaceutically acceptable additive.

8. The combination according to any one of claims 1 to 6 or the pharmaceutical composition according to claim 7 for the treatment of a cancer.

9. The combination or the pharmaceutical composition according to claim 8, wherein the cancer expresses BcL-XL.

10. The combination or the pharmaceutical composition according to claim 8, wherein the cancer is selected from the group consisting of brain tumor, head and neck cancer, breast cancer, lung cancer, oral cancer, esophageal cancer, stomach cancer, duodenal cancer, appendix cancer, colon cancer, rectal cancer, liver cancer, pancreatic cancer, gallbladder cancer, bile duct cancer, anal cancer, kidney cancer, ureteral cancer, bladder cancer, prostate cancer, penile cancer, testicular cancer, uterine cancer, cervical cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and leukemia.

11. A method for treating a cancer, comprising administering an effective amount of a combination according to any one of claims 1 to 6 and 8 to 10 or a pharmaceutical composition according to any one of claims 7 to 10 to a subject in need thereof.

12. A pharmaceutical composition for treating a cancer in combination with an anticancer agent, the pharmaceutical composition comprising a molecule comprising the nucleotide sequence of SEQ ID NO: 1.


SEQUENCE LISTING

<110>  Nitto Denko Corporation

<120>  Combination for use in cancer treatment

<130>  G2071WO

<150>  JP 2018-228283
<151>  2018-12-05

<160>  6

<170>  PatentIn version 3.5

<210>  1
<211>  7
<212>  RNA
<213>  Artificial sequence

<220>
<223>  CUGACUC sequence

<400>  1
cugacuc                                                                 7


<210>  2
<211>  22
<212>  RNA
<213>  Homo sapiens

<400>  2
gcugacuccu aguccagggc uc                                               22


<210>  3
<211>  98
<212>  DNA
<213>  Homo sapiens

<400>  3
acccaaaccc taggtctgct gactcctagt ccagggctcg tgatggctgg tgggccctga      60

acgaggggtc tggaggcctg ggtttgaata tcgacagc                              98


<210>  4
<211>  21
<212>  DNA
<213>  Artificial sequence

<220>
<223>  Compound A sense strand


<220>
<221>  misc_feature
<223>  Combined DNA/RNA molecule

<400>  4
gguauuggug agucggauct t                                                21

<210>  5
<211>  21
<212>  DNA
<213>  Artificial sequence

<220>
<223>  Compound A antisense strand


<220>
<221>  misc_feature
<223>  Combined DNA/RNA molecule

<400>  5
gauccgacuc accaauacct t                                                21


<210>  6
<211>  23
<212>  DNA
<213>  Artificial sequence

<220>
<223>  BcL-XL-specific gRNA

<400>  6
gtttgaactg cggtaccggc ggg                                              23